# EUROPÄISCHE PATENTANMELDUNG

(11) **EP 4 567 020 A1**
(43) Veröffentlichungstag der Anmeldung: **11.06.2025**
(21) Anmeldenummer: 23214551.6
(22) Anmeldetag: 06.12.2023
(51) Int. Cl.: C07C 29/80, C07C 31/20, C09K 5/20

(54) **REINIGUNG VON KÜHLMITTELZUSAMMENSETZUNGEN**

(71) Anmelder: BASF SE, 67056 Ludwigshafen am Rhein (DE)
(72) Erfinder: MALKOWSKY, Itamar Michael, 67056 Ludwigshafen am Rhein (DE); WENDLING, Timo, 67056 Ludwigshafen am Rhein (DE); KRKLJUS, Ivana, 67056 Ludwigshafen am Rhein (DE); MAYER, Guido, 67056 Ludwigshafen am Rhein (DE); SCHNELL, Sandra, 67056 Ludwigshafen am Rhein (DE); SCHWAB, Tobias Bastian, 67056 Ludwigshafen am Rhein (DE)
(74) Vertreter: BASF IP Association

(57) **Zusammenfassung**

Die vorliegende Erfindung beschreibt ein Verfahren zur Reinigung gebrauchter Kühlmittelzusammensetzungen und ein Verfahren zur Senkung von Emissionen durch Reinigung gebrauchter Kühlmittelzusammensetzungen.

## Beschreibung

Die vorliegende Erfindung beschreibt ein Verfahren zur Reinigung gebrauchter Kühlmittelzusammensetzungen und ein Verfahren zur Senkung von Emissionen durch Reinigung gebrauchter Kühlmittelzusammensetzungen.

Bei Kühlmitteln zur Anwendung in mobilen oder stationären Verbrennungsmotoren, aber auch in Kühlkreisläufen von Elektrofahrzeugen oder Kombinationen von Fahrzeugen mit Elektro- und Verbrennungsmotoren handelt es sich im allgemeinen um flüssige Mischungen aus Wasser, Glykolen, Korrosionsinhibitoren und weiteren Bestandteilen.

Bei der gefrierpunktserniedrigenden Glykolkomponente handelt es sich dabei zumeist um Monoethylenglykol und/oder Monopropylenglykol, überwiegend Monoethylenglykol.

Im Rahmen dieser Schrift werden Monoethylenglykol und/oder Monopropylenglykol zusammenfassend als "Glykole" bezeichnet, dabei kann es sich um Monoethylenglykol oder Monopropylenglykol oder Gemische davon handeln, bevorzugt handelt es sich jedoch um Monoethylenglykol.

Industriell wird Monoethylenglykol üblicherweise durch Öffnung von Ethylenoxid mit Wasser hergestellt, wobei das Ethylenoxid aus Ethen hergestellt wird, das wiederum durch Spaltung von fossilem Naphtha in Steamcrackern erhalten wird. Ein weiterer Herstellungsprozeß ist der sogenannte Omega-Prozeß, in dem Ethylenoxid mit Kohlenstoffdioxid zunächst zum cyclischen Carbonat umgesetzt wird, das dann anschließend mit Wasser zum Monoethylenglykol geöffnet wird. Auch hier stammt das Ethylenoxid meist aus fossilen Quellen.

Dieser Herstellungsweg hat zumindest die folgenden Nachteile:
- Bei fossilen Rohstoffen handelt es sich um eine endliche Ressource
- Der Einsatz fossiler Rohstoffe erhöht den Carbon-Footprint und führt zu einer schlechten Ökobilanz der erhaltenen Produkte

In der Praxis seltener wird Monopropylenglykol (1,2-Propylenglykol) anstelle Monoethylenglykol als gefrierpunktserniedrigende Glykolkomponente eingesetzt. Die Problematik ist jedoch im wesentlichen die gleiche, da 1 ,2-Propylenglykol analog aus Propylenoxid hergestellt wird, dieses wiederum aus Propen, das ebenfalls aus Naphtha gewonnen wird.

Die wässrigen Kühlmittel werden während des Betriebes in Kühlkreisläufen thermisch belastet und zersetzen sich während der Betriebsdauer zunehmend. Dabei bilden sich zumeist Oxidationsprodukte, im Fall von Monoethylenglykol beispielsweise Glykolsäure (HO-CH₂-COOH), Glyoxylsäure (OHC-COOH), Oxalsäure (HOOC-COOH), Glyoxal (OHC-CHO) und/oder Glykolaldehyd (HO-CH₂-CHO), aber auch ein Kettenabbau zu C₁-Verbindungen (Formaldehyd und/oder Ameisensäure). Dies führt zu einer starken Absenkung des pH-Wertes gebrauchter Kühlmittel unter pH 7, teilweise unter pH 6,5 und sogar bis zu pH-Werte von 6.

Sobald das Kühlmittel durch diese Veränderung seine Funktion nicht mehr erfüllt oder auch im Rahmen regulärer Wechselintervalle, z.B. Service oder Inspektion, wird das gebrauchte Kühlmittel aus dem Kühlkreislauf entfernt. Da Monoethylenglykol nach OECD 301 Tests biologisch leicht abbaubar klassifiziert ist, wird das gebrauchte Kühlmittel meist per Abwasser über eine Kläranlage entsorgt.

Das gebrauchte Kühlmittel enthält neben Wasser als Hauptmenge immer noch Monoethylenglykol, in der Regel werden von dem ursprünglich eingesetzten Monoethylenglykol nicht mehr als 20%, meist sogar nicht mehr als 10%, bevorzugt nicht mehr als 5% zersetzt. Dennoch muss das gebrauchte Kühlmittel ausgetauscht werden, da durch den Abbau an Monoethylenglykol seine Fähigkeit zur Gefrierpunktserniedrigung abnimmt, durch die Anwesenheit der Säuren unter den Abbauprodukten sein Korrosivität zunimmt und/oder durch die Bildung von aldehydischen Verbindungen vermehrt Polymere entstehen, die sich abscheiden können und an den zu kühlenden Flächen durch Ablagerungen den Wärmeübergangskoeffizient verschlechtern bzw. Kanäle des Wärmetauschers im Kühlkreislauf verstopfen können. Die Bildung derartiger Polymere lässt sich leicht anhand der oftmals gelb- bis bräunlichen Farbe des gebrauchten Kühlmittels erkennen.

Wenn diese gebrauchten Kühlmittel also gesammelt und zentral aufgearbeitet würden, so könnte das Glykol zumindest teilweise einer weiteren stofflichen Verwertung zugeführt werden.

Ein weiterer Nachteil der Zusammensetzung gebrauchter Kühlmittel der eine Wiedergewinnung erschwert und insbesondere aus der Kombination aus Säuren mit Ethylenglykol resultiert, ist die Bildung von Dioxan unter Einwirkung von Säure bei erhöhter Temperatur, da unter diesen Bedingungen verstärkt die Reaktion von Ethylenglykol zu Diethylenglykol und Ringschluss zu Dioxan abläuft.

Da für Dioxan eine krebserzeugende Wirkung in Betracht gezogen wird, ist dessen Gehalt so gering wie möglich zu halten.

Aufgabe der vorliegenden Erfindung war es, ein Verfahren zur Verfügung zu stellen, mit dem Glykole aus gebrauchten Kühlmitteln wiedergewonnen werden können.

Demgemäß wurde ein Verfahren zur Reinigung gebrauchter, wäßriger glykolhaltiger Kühlmittel gefunden, in dem man
- zunächst in einem Schritt (a) von dem gebrauchten, wäßrigen glykolhaltigen Kühlmittel destillativ leichter als Glykol siedende Inhaltsstoffe bei einem Druck von 50 mbar bis Normaldruck und einer Temperatur von 50 bis 140 °C abtrennt und
- anschließend in einem Schritt (b) aus dem Destillationsrückstand des Schrittes (a) das Glykol bei einem Druck von 50 mbar bis Normaldruck und einer Temperatur von 50 bis 140 °C abdestilliert.

Unter diesen Trennungsbedingungen erhält man ein Destillat, in dem die Nebenkomponenten ausreichend abgereichert sind, dass man das erhaltene Glykol von Neuem für Kühlmittel einsetzen kann. Die Destillationstemperaturen sind einerseits so niedrig gewählt, dass während der Destillation keine signifikante Zersetzung oder weitere Reaktion des Glykols festgestellt wird und die Destillation andererseits mit industriell verfügbaren Vakuumanlagen betrieben werden kann. Extreme Destillationstemperaturen sind nicht erforderlich.

Als weiterer Gegenstand der vorliegenden Erfindung wurde ein Verfahren zur Reinigung gebrauchter, wäßriger glykolhaltiger Kühlmittel gefunden, umfassend mindestens die Schritte
(a) destillative Abtrennung von leichter als Glykol siedenden Inhaltsstoffen,
(b) Destillation von Glykol,
   sowie darüberhinaus noch mindestens einen der Schritte
(c) Strippen eines glykolhaltigen Gemischs mit einem Gas, bevorzugt einem Inertgas,
(d) Behandlung eines glykolhaltigen Gemischs mit einem sauren oder basischen, bevorzugt basischen Feststoff,
(e) Behandlung eines glykolhaltigen Gemischs mit Aktivkohle, und/oder
(f) Membranfiltration eines glykolhaltigen Gemischs.

Die Destillation von Glykolen, besonders Monoethylenglykol, ist aus dem Herstellungsprozess des Monoethylenglykol aus Ethylenoxid mit Wasser zwar schon an sich bekannt, jedoch ist diese destillative Aufarbeitung nicht ohne weiteres auf gebrauchte Kühlmittel übertragbar, da die Nebenproduktspektren und somit die Trennungsaufgaben der Destillationen unterschiedlich sind:
Bei der Umsetzung von Ethylenoxid mit Wasser entsteht ein Reaktionsgemisch an, das im wesentlichen Wasser, Monoethylenglykol und dessen höheren Oligomere, beispielsweise Diethylenglykol, Triethylenglykol, Tetraethylenglykol usw. enthält. Das Verhältnis der einzelnen Oligomere zueinander wird dabei durch das eingesetzte Verhältnis von Wasser zu Ethylenoxid bestimmt. Die Trennungsaufgabe besteht also darin, Wasser und die einzelnen Oligomere möglichst rein voneinander zu trennen, wobei die Destillationsbedingungen so gewählt sein sollten, dass die Glykole nicht rückspalten, also z.B. durch Eliminierung von Wasser oder Abspaltung von Ethylenoxid. Daher wird meist die Destillationstemperatur möglichst gering gewählt.

Demgegenüber besteht in gebrauchten Kühlmitteln die Hauptmenge aus Wasser, höhere Oligomere des Monoethylenglykol sind in der Regel, wenn überhaupt, in nicht signifikanten Mengen enthalten. Dafür enthalten die gebrauchten Kühlmittel deutliche Mengen der oben aufgeführten C₁- und C₂- Abbauprodukte, besonders von Säuren und/oder Aldehyden.

Typischerweise sind die gebrauchten Kühlmittel wie folgt zusammengesetzt:
- Wasser: 50 bis 75 Gew%
- Glykol: 25 bis 50 Gew%
- höhere Glykololigomere: 0,1 bis 3 Gew%
- C₁-Abbauprodukte: 0,1 bis 2 Gew%
- C₂-Abbauprodukte: 0,1 bis 2 Gew%
- höhere organische Säuren und deren Abbauprodukte: 0,1 bis 5 Gew%
- anorganische Bestandteile: 0,1 bis 3 Gew%
- andere Bestandteile: bis zu 5 Gew%
mit der Maßgabe, dass die Summe immer 100 Gew% beträgt.

Bei dem Glykol handelt es sich zumeist um Monoethylenglykol, Monopropylenglykol und/oder Glycerin, bevorzugt Monoethylenglykol. Da bei einem Austausch z.B. in Werkstätten verschiedene gebrauchte Kühlmittel vereinigt werden können, handelt es sich bei dem Glykol nicht notwendigerweise ein einheitliches Glykol, was anzustreben wäre, sondern es können durchaus Gemische verschiedener Glykole vorliegen, beispielsweise Monoethylenglykol, Monopropylenglykol und Glycerin, Monoethylenglykol und Glycerin oder Monoethylenglykol und Monopropylenglykol.

Bei einem Gemisch von Glykolen ist Monoethylenglykol zumeist die Hauptkomponente und ist unter den Glykolen zu mindestens 80, bevorzugt mindestens 90, besonders bevorzugt mindestens 95 Gew% enthalten. Die Differenz zu 100 Gew% bilden dann Monopropylenglykol und Glycerin, meist Monopropylenglykol.

Bei den höheren Glykololigomeren handelt es sich um die Oligomere der oben genannten Glykole mit mindestens 2 Wiederholungseinheiten, besonders Diethylenglykol, Triethylenglykol, Tetraethylenglykol, usw, sowie weniger bevorzugt. Dipropylenglykol, Tripropylenglykol, Tetrapropylenglykol. Diese können in geringem Ausmaß bereits von vornherein in dem Kühlmittel enthalten sein oder entstehen während des Betriebs des Kühlmittels durch Etherbildung unter den Bedingungen im Kühlkreislauf.

Eine weitere typische Quelle für diese höheren Oligomere sind versehentliche Verunreinigungen der gebrauchten Kühlmittel mit Bremsflüssigkeiten, die üblicherweise diese Oligomere, deren Monoalkylether und/oder deren Borsäureester enthalten.

C₁-Abbauprodukte sind bevorzugt Formaldehyd und/oder Ameisensäure.

C₂-Abbauprodukte sind Glykolsäure (HO-CH₂-COOH), Glyoxylsäure (OHC-COOH), Oxalsäure (HOOC-COOH), Glyoxal (OHC-CHO), Glykolaldehyd (HO-CH₂-CHO), Acetaldehyd und/oder Essigsäure.

Höhere organische Säuren, d.h. mindestens 3 Kohlenstoffatome aufweisende Mono- und Dicarbonsäuren, sind in neuen Kühlmitteln häufig als Korrosionsinhibitoren gegen Metallkorrosion, beispielsweise von Eisenwerkstoffen, Aluminium, Buntmetallen oder Lot, enthalten und finden sich demzufolge zusammen mit deren Abbauprodukten auch in den gebrauchten Kühlmitteln.

Bevorzugte Monocarbonsäuren weisen 5 bis 12 Kohlenstoffatome auf, besonders bevorzugt 6 bis 10, ganz besonders bevorzugt 8, 9 oder 10.

Bevorzugte Individuen sind Pentansäure, 2,2-Dimethylpropansäure, Hexansäure, 2,2-Dimethylbutansäure, Octansäure, 2-Ethylhexansäure, Nonansäure, Isononansäure, Decansäure, Undecansäure und Dodecansäure, sowie deren Isomerengemische, insbesondere 2-Ethylhexansäure und Isononansäure-Isomerengemische.

Die Monocarbonsäuren sind zumeist aliphatisch, es kann aber auch Benzoesäure als Monocarbonsäure enthalten sein.

Bevorzugte Dicarbonsäuren sind 4 bis 20, bevorzugt 5 bis 14 und besonders bevorzugt 6 bis 12 Kohlenstoffatome aufweisende lineare oder verzweigte Alkandicarbonsäuren, bevorzugt lineare Alkan- oder Alkendicarbonsäuren, besonders bevorzugt Alkandicarbonsäuren.

Bevorzugt sind die Dicarbonsäuren ausgewählt aus der Gruppe bestehend aus Bernsteinsäure, Maleinsäure, Fumarsäure, Glutarsäure, Adipinsäure, Pimelinsäure (Heptandisäure), Azelainsäure (Nonandisäure), Sebacinsäure (Dekandisäure), Undekandisäure, Dodekandisäure, sowie Alkyl- und Alkenylbernsteinsäuren und -glutarsäuren wie 2-Methylbutandisäure, 2-ethyl-3-methylbutandisäure, 2-Ethylpentandisäure, 2-Dodecylbutandisäure, 2-Dodecenylbutandisäure, 2-Phenylbutandisäure, 2-(p-Methyl phenyl) butandisäure, 2,2-Dimethylbutandisäure, 2,3,4-Trimethylpentandisäure, 2,2,3-Trimethylpentandisäure, Glutaconsäure (Pent-2-endisäure), Itaconsäure, Hex-2-endisäure, Hex-3-endisäure, 5-Methyl-hex-2-endisäure und 2,3-Diemethyl-pent-2-endisäure.

Eine weitere Quelle für höhere organische Säuren können aus auch Beimischungen von Bremsflüssigkeiten (siehe unten) stammen, in denen beispielsweise Laurinsäure, Palmitinsäure, Stearinsäure oder Ölsäure als Korrosionsinhibitoren enthalten sein können.

Anorganische Bestandteile umfassen bevorzugt Silikate, Borate, Nitrate, Molybdate, Vanadate und Phosphate, die überwiegend als Inhibitor der Korrosion von Aluminium wirken.

Die Silikate sind bevorzugt ausgewählt aus der Gruppe bestehend aus Orthosilikaten (SiO₄⁴⁻), Metasilikaten (SiO₃²⁻), und Pyrosilikaten (Si₂O₇⁶⁻), besonders bevorzugt handelt es sich um Metasilikate (SiO₃²⁻), ganz besonders bevorzugt um Natriummetasilikat (Na₂SiO₃) oder Kaliummetasilikat (K₂SiO₃), insbesondere um Natriummetasilikat (Na₂SiO₃).

Ferner können sich Silikate auch durch Hydrolyse von ortho-Kieselsäureestern bilden, beispielsweise Tetramethoxysilan und Tetraethoxysilan.

Die Borate werden bevorzugt als Natriumtetraborat (Borax) oder als Kaliumtetraborat eingesetzt, besonders bevorzugt als Natriumtetraborat.

Die Nitrate werden als Alkali- oder Erdalkalimetallnitrate eingesetzt, bevorzugt als Natriumnitrat, Kaliumnitrat oder Magnesiumnitrat, bevorzugt als Natriumnitrat oder Kaliumnitrat, besonders bevorzugt als Natriumnitrat.

Die Phosphate werden als freie Säure (H₃PO₄), als Hydrogenphosphat, Dihydrogenphosphat oder Phosphat eingesetzt, bevorzugt als Natrium oder Kaliumsalz.

Denkbar ist auch der Einsatz der entsprechenden Diphosphate, Triphosphate oder Oligophosphate, bevorzugt werden sie jedoch als monomere Phosphate eingesetzt.

Bevorzugt ist der Einsatz als freie Säure (H₃PO₄), Dinatriumhydrogenphosphat oder Trinatriumphosphat.

Andere Bestandteile sind solche, die nicht unter eine der obigen Kategorien fallen. Dabei handelt es sich überwiegend um unidentifizierbare Bestandteile der gebrauchten Kühlmittel, um versehentliche Verunreinigungen oder intelligente Fehlwürfe.

Denkbar ist z.B. in Werkstätten eine Verunreinigung mit Ölen, beispielsweise Motor- oder Getriebeöl oder aus Bremsflüssigkeiten.

Aus letzteren können beispielsweise höhere Ethylenglykole und -glykolether stammen, beispielsweise Di-, Tri- und Tetraethylengylkol sowie Di-, Tri- und Tetraethylengylkol monoalkylether, wobei es sich bei den Alkylethern meist um Methyl-, Ethyl- oder n-Butylether handelt. Ebenfalls aus Bremsflüssigkeiten stammen die Borsäureester dieser Ethylenglykole und -glykolether, sowie Amine, besonders Monoethanolamin, Diethanolamin, Triethanolamin, Diisopropanolamin, Diisopropylamin und Alkyldiethanolamine, wobei es sich bei Alkyl hier meist C₄- bis C₁₂-Alkyl handelt, besonders Butyl oder Octyl. Quellen der Amine als Bestandteil in gebrauchten Kühlmitteln können sowohl Bremsflüssigkeiten als auch Kühlmittel sein. Möglich ist auch der Eintrag von Phosphorverbindungen, besonders Estern der Phosphorigen oder Phosphorsäure, beispielsweise Ethylphosphat, Dimethylphosphat, Isopropylphosphat, n-Butylphosphat, Triphenylphosphit oder Diisopropylphosphit.

An der Vielzahl und Natur der anderen Bestandteile der gebrauchten Kühlmittel neben den gewünschten Glykolen ist leicht ersichtlich, dass sich die Trennungsaufgabe deutlich von der aus dem Herstellungsprozess des Monoethylenglykol aus Ethylenoxid und Wasser unterscheidet, da andere Komponenten im gebrauchten Kühlmittel enthalten sein können und dazu auch noch in Mengen und Verhältnissen, die aus der Herstellung von Monoethylenglykol aus Ethylenoxid und Wasser nicht bekannt sind.

Ein Gegenstand der vorliegenden Erfindung ist ein Verfahren zur Reinigung gebrauchter, wäßriger glykolhaltiger Kühlmittel, umfassend mindestens die Schritte
(a) destillative Abtrennung von leichter als Glykol siedenden Inhaltsstoffen,
(b) Destillation von Glykol,
   sowie darüberhinaus noch mindestens einen der Schritte
(c) Strippen eines glykolhaltigen Gemischs mit einem Gas, bevorzugt einem Inertgas,
(d) Behandlung eines glykolhaltigen Gemischs mit einem sauren oder basischen, bevorzugt basischen Feststoff,
(e) Behandlung eines glykolhaltigen Gemischs mit Aktivkohle, und/oder
(f) Membranfiltration eines glykolhaltigen Gemischs.

Die einzelnen Schritte seien im folgenden näher erläutert:
Die gebrauchten Kühlmittel werden zunächst dezentral gesammelt, beispielsweise in Werkstätten. Die Kühlmittel können aus Kraftfahrzeugen stammen, bei denen beispielsweise im Rahmen einer Inspektion ein teilweiser oder vollständiger Austausch des enthaltenen Kühlmittels vorgenommen wird. Üblicherweise wird nach dem Ablassen des gebrauchten Kühlmittels das Kühlsystem mit Wasser gespült, um Reste oder etwaige Rückstände oder Ablagerungen zu entfernen, so dass das gebrauchte Kühlmittel einen höheren Wassergehalt aufweist als das üblicherweise eingesetzte betriebsbereite neue Kühlmittel.

Weiterhin können gebrauchte Kühlmittel im Rahmen einer Wartung von Kühlsystemen ortsfester Motoren anfallen, beispielsweise Brennstoffzellen, Aggregate, Generatoren oder Windkraftanlagen. Beschrieben ist auch die Anwendungen zum Wärmemanagement in von elektronischen Geräten, beispielsweise in Datenzentren, wie z.B. gemäß WO 2023/06584 A1.

Die dabei anfallenden gebrauchten Kühlmittel sind in der Regel nicht nach deren Inhaltsstoffen getrennt, sondern stellen ein Gemisch der marktüblichen in Kühlmitteln eingesetzten Inhaltsstoffe mit variabler Zusammensetzung dar.

Die so angefallenen gebrauchten Kühlmittel werden dann von den Werkstätten und Wartungsfirmen gesammelt und vereinigt und zur Aufarbeitung im erfindungsgemäßen Verfahren transportiert.

Falls erforderlich können die wässrigen gebrauchten Kühlmittel vor Einsatz in das erfindungsgemäße Verfahren einer Phasenabscheidung unterzogen werden. Dies kann beispielsweise dann der Fall sein, wenn das gesammelte Material mit Öl verunreinigt ist, so dass die abgeschiedene organische Phase in einem Ölabscheider separiert wird. Zusätzlich oder stattdessen kann auch ein etwaiger Feststoff durch Filtration, Sedimentieren oder Dekantieren abgetrennt werden. Feststoffe können sich beispielsweise bilden oder enthalten sein durch versehentliche Verunreinigungen durch Fremdstoffe oder auch durch Reaktion von Kühlmitteln unterschiedlicher Zusammensetzung, beispielsweise durch Ausfallen der Reaktionsprodukte saurer und basischer Bestandteile oder ausgefällten anorganischen Bestandteilen.

Erfindungsgemäß werden die Schritte
(a) destillative Abtrennung von leichter als Glykol siedenden Inhaltsstoffen und
(b) Destillation von Glykol
   durchlaufen, sowie zusätzlich noch mindestens einer der Aufarbeitungsschritte
(c) Strippen eines glykolhaltigen Gemischs mit einem Gas, bevorzugt einem Inertgas,
(d) Behandlung eines glykolhaltigen Gemischs mit einem sauren oder basischen, bevorzugt basischen Feststoff,
(e) Behandlung eines glykolhaltigen Gemischs mit Aktivkohle, und/oder
(f) Membranfiltration eines glykolhaltigen Gemischs.

### (a) Destillative Abtrennung von leichter als Glykol siedenden Inhaltsstoffen

Im Schritt (a) werden im wesentlichen die Leichtsieder, hier also leichter als Glykol siedenden Bestandteile, des gebrauchten Kühlmittels entfernt, besonders Wasser, leichtsiedende organische Säuren, wie Ameisensäure oder Essigsäure, aldehydische Bestandteile, besonders Formaldehyd, Acetaldehyd und deren höheren Oligomere, wie Crotonaldehyd, Pentadienal, Hexadienal usw., und die oben aufgeführten C₂-Abbauprodukte. Dieser Leichtsiederstrom wird im folgenden als (a1) bezeichnet.

Die destillative Abtrennung dieser Leichtsieder erfolgt bevorzugt kontinuierlich und beispielsweise in einem Rührkessel mit Doppelwandheizung und/oder innenliegenden Heizschlangen unter vermindertem Druck, beispielsweise von 50 mbar bis Normaldruck, bevorzugt 100 bis 900 mbar und besonders bevorzugt 200 bis 800 mbar (absolut) und einer Temperatur (Kopftemperatur) von 50 bis 140 °C, bevorzugt 60 bis 130 und besonders bevorzugt 70 bis 120 °C.

Selbstverständlich kann die Destillation auch in einem Fallfilm-, Dünnschicht- oder Wischblattverdampfer erfolgen. Dazu wird das wässrige Gemisch, kontinuierlich oder diskontinuierlich, unter vermindertem oder normalem Druck, beispielsweise von 50 mbar bis Normaldruck, bevorzugt 100 bis 900 mbar und besonders bevorzugt 200 bis 800 mbar (absolut) und einer Temperatur (Kopftemperatur) von 50 bis 140 °C, bevorzugt 60 bis 130 und besonders bevorzugt 70 bis 120 °C durch den Apparat geführt.

Vorteilhaft kann ein inertes Gas, bevorzugt Argon oder ein stickstoffhaltiges Gas, besonders bevorzugt Argon, Stickstoff oder ein Gemisch aus Luft und Stickstoff (Magerluft), ganz besonders bevorzugt Stickstoff in den Destillationsapparat eingeleitet werden, beispielsweise 0,1 - 1, bevorzugt 0,2 - 0,8 und besonders bevorzugt 0,3 - 0,7 m³/m³h, bezogen auf das Volumen des flüssigen Gemisches.

In einer bevorzugten Ausführungsform kann als inertes Gas Wasserdampf als Strippstrom eingesetzt werden.

Im Schritt (a) wird der Wassergehalt des eingesetzten gebrauchten Kühlmittels in der Regel um mindestens ein Drittel des ursprünglichen Gehalts verringert, bevorzugt um mindestens die Hälfte und besonders bevorzugt um mindestens zwei Drittel.

Die oben aufgeführten nichtwässrigen Leichtsieder werden in der Regel mindestens um die Hälfte des ursprünglichen Gehalts verringert, bevorzugt um mindestens zwei Drittel und besonders bevorzugt um mindestens drei Viertel.

Weiterhin kann das Destillat auch in untergeordneten Mengen Glykol enthalten.

Die so im Destillat (a1) abgetrennten Leichtsieder werden kondensiert und bevorzugt in eine Kläranlage zum Abbau der nichtwässrigen organischen Bestandteile geführt.

Der verbliebene Destillationsrückstand (a2) enthält überwiegend Glykol und Hochsieder, hier also höher als Glykol siedenden Bestandteile. Bevorzugt wird dieser Destillationsrückstand direkt und bei der bestehenden Temperatur in den Schritt (b) geführt.

Es kann sinnvoll sein, die Destillationsbedingungen in Schritt (a) in Abhängigkeit des pH-Wertes des Destillationsrückstandes (a2) zu regeln, um zusammen mit dem Leichtsiederstrom (a1) leichtsiedende saure Inhaltsstoffe zu entfernen. Wenn im Destillationsrückstand (a2) eine bestimmte pH-Schwelle unterschritten wird, so können beispielsweise in der Destillation die Temperatur erhöht und/oder der Druck abgesenkt und/oder der Strippstrom verstärkt werden. Eine derartige Schwelle ist beispielsweise ein pH-Wert von 7, dessen Unterschreitung im Strom (a2) das Vorhandensein größerer Mengen von Säuren anzeigt, die destillativ abgetrennt werden können.

### (b) Destillation von Glykol

Im Schritt (b) wird das Glykol im wesentlichen reindestilliert. In der Regel werden in diesem Destillationsschritt drei Fraktionen erhalten, eine überwiegend Wasser und Glykol enthaltende (b1), eine Glykolfraktion (b2), sowie ein Hochsiederstrom (b3).

In einer bevorzugten Ausführungsform kann der Schritt (b) zwei Rektifikationskolonnen umfassen, in deren erster die überwiegend Wasser und Glykol enthaltende Fraktion (b1) über Kopf abgetrennt wird, der Destillationsrückstand aus Glykol und Hochsieder dann in die zweite Rektifikationskolonne geführt wird, in der die Glykolfraktion (b2) über Kopf abgetrennt wird und der Destillationssumpf die Hochsiederfraktion (b3) bildet.

Denkbar ist auch die Durchführung der Destillation in einer Trennwandkolonne anstatt zweier getrennter Rektifikationskolonnen.

Denkbar, wenn auch weniger bevorzugt ist eine Ausführungsform, in der in der ersten Rektifikationskolonne Wasser und Glykol vollständig über Kopf genommen werden und vom Destillationssumpf (b3) getrennt werden. Der Brüden aus Wasser und Glykol wird dann in einer zweiten Rektifikationskolonne in die Fraktionen Wasser und Glykol (b1) über Kopf und die Glykolfraktion (b2) im Sumpf aufgespalten.

In einer weiteren bevorzugten Ausführungsform werden die drei Fraktionen (b1), (b2) und (b3) in einer einzelnen Rektifikationskolonne aufgetrennt, Wasser und Glykol als Kopffraktion (b1), die Glykolfraktion (b2) im Seitenabzug und die Hochsieder im Sumpf (b3).

Die Destillationseinheiten sind von an sich bekannter Bauart. Als Kolonneneinbauten der Rektifikationskolonnen kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern oder Geflechten bevorzugt.

Umlaufverdampfer und Kondensatoren der Rektifikationskolonnen sind ebenfalls von herkömmlicher Bauart, bevorzugte Beispiele sind Rohrbündelwärmetauscher und Plattenwärmetauscher.

Im Fall der Ausführungsformen mit zwei getrennten Rektifikationskolonnen weisen diese meist jeweils von 10 bis 40, bevorzugt 15 bis 35, besonders bevorzugt 20 bis 30 theoretische Trennböden auf.

Im Fall der Ausführungsform der Abtrennung in einer Rektifikationskolonne weist diese 10 bis 60, bevorzugt 15 bis 50, besonders bevorzugt 20 bis 40 theoretische Trennböden auf.

Weniger theoretische Trennböden erbringen zumeist nicht die erforderlich Trennleistung, mehr theoretische Trennböden erbringen zumeist keinen weiteren Vorteil in der Aufreinigung.

Die Destillationsvorrichtungen werden in der Regel bei 5 - 500 mbar, bevorzugt 10 bis 300 mbar, besonders bevorzugt 50 bis 200 mbar (absolut) betrieben und einer Kopftemperatur von 80 bis 180 °C, bevorzugt 100 bis 160 °C.

Vorteilhaft kann ein inertes Gas, bevorzugt Argon oder ein stickstoffhaltiges Gas, besonders bevorzugt Argon, Stickstoff oder ein Gemisch aus Luft und Stickstoff (Magerluft), ganz besonders bevorzugt Stickstoff in den Destillationsapparat eingeleitet werden, beispielsweise 0,1 - 1, bevorzugt 0,2 - 0,8 und besonders bevorzugt 0,3 - 0,7 m³/m³h, bezogen auf das Volumen des flüssigen Gemisches.

Die in diesem Schritt als Destillat (b1) abgetrennte Fraktion aus Glykol und Wasser wird kondensiert und kann in eine Kläranlage zum Abbau der nichtwässrigen organischen Bestandteile oder in eine Rückstandsverbrennung geführt werden. Im Falle eines niedrigen Wassergehaltes bzw. eines hohen Brennwerts ist eine Verbrennung bevorzugt, andernfalls ein biologischer Abbau in einer Kläranlage.

Der im Schritt (b) verbliebene Destillationsrückstand (b3) wird bevorzugt einer Rückstandsverbrennung zugeführt.

Eine bevorzugte Ausführungsform der vorliegenden Erfindung stellt also die oben beschriebene Verschaltung der Schritte (a) und (b) dar, in der in der Stufe (a) überwiegend Wasser als Fraktion (a1) entfernt wird und in Schritt (b) das Glykol im Seitenabzug (b2) erhalten wird neben einer Wasser und Glykol enthaltenden Fraktion (b1) als Kopfabzug sowie einem Destillationsrückstand (b3). Diese Ausführungsform ist besonders dann bevorzugt, wenn der Austrag (a2) aus dem Schritt (a) einen Wassergehalt von 2 Gew% oder mehr aufweist.

Die Abnahme des Glykol im Seitenabzug (b2) ist auch dann bevorzugt, wenn bei der hohen Sumpftemperatur signifikant Dioxan gebildet wird, also besonders unter sauren Bedingungen.

Bei niedrigeren Wassergehalten des Austrags (a2) aus Schritt (a), bevorzugt bei nicht mehr als 1,5 Gew%, besonders bevorzugt nicht mehr als 1 und ganz besonders bevorzugt nicht mehr als 0,5 Gew%, kann es bevorzugt sein, in Schritt (b) auf die Abtrennung der Wasser und Glykol enthaltenden Fraktion (b1) zu verzichten, sondern stattdessen die Glykolfraktion (b2) in Schritt (b) über Kopf abzunehmen.

Aus energetischen Gründen kann es vorteilhaft sein, die Rektifikationskolonne in Schritt (b) bei einem niedrigeren Druck als die Kolonne in Stufe (a) zu betreiben, der ausreicht, um die verdampfbaren Komponenten in dem Strom (a2) bei dessen Austragstemperatur zu verdampfen und die Kolonne (b) so ohne weitere Erhitzung dieses Stroms zu betreiben.

Die unabhängig von der Ausführungsform erhaltene, überwiegend das Wertprodukt Glykol enthaltende Fraktion (b2) ist meist wie folgt zusammengesetzt:
- Wasser: nicht mehr als 5 Gew%, bevorzugt nicht mehr als 3, besonders bevorzugt nicht mehr als 1 Gew%,
- Glykol: 90 bis 99,9 Gew%, bevorzugt 95 bis 99, 9, besonders bevorzugt 98 bis 99,8 Gew%
- höhere Glykololigomere: 0,01 bis 2 Gew%, bevorzugt 0,01 bis 1, besonders bevorzugt 0,01 bis 0,5 Gew%,
- C₁-Abbauprodukte: nicht mehr als 0,25 Gew%, bevorzugt nicht mehr als 0,1, besonders bevorzugt nicht mehr als 0,05 Gew%
- C₂-Abbauprodukte: nicht mehr als 0,5 Gew%, bevorzugt nicht mehr als 0,25, besonders bevorzugt nicht mehr als 0,1 Gew%
- höhere organische Säuren und deren Abbauprodukte: nicht mehr als 1 Gew%, bevorzugt nicht mehr als 0,5, besonders bevorzugt nicht mehr als 0,25 Gew%
- anorganische Bestandteile: nicht mehr als 0,1 Gew%
- andere Bestandteile: nicht mehr als 0,5 Gew%
mit der Maßgabe, dass die Summe immer 100 Gew% beträgt.

Falls gewünscht kann die Fraktion (b2) anschließend nochmals einer weiteren Rektifikation unterworfen werden, in der Glykol von Hochsiedern und/oder Leichtsiedern abgetrennt wird, falls die Reinheit des Glykols noch nicht den Ansprüchen genügt.

Destillative Trennverfahren zur Aufreinigung von Wasser-Ethylenglykol-Gemischen aus der Hydrolyse von Ethylenoxid sind bekannt, siehe z.B. WO 00/17140. Die darin beschriebene Trennaufgabe besteht jedoch vorwiegend in der Abtrennung von Aldehyden, besonders Formaldehyd, Acetaldehyd, Crotonaldehyd und Glykolaldehyd.

Säuren spielen in dieser Trennaufgabe keine oder allenfalls eine untergeordnete Rolle, so dass die Lehre der Aufreinigung von Wasser-Ethylenglykol-Gemischen aus der Hydrolyse von Ethylenoxid nicht ohne weiteres auf die vorliegenden gebrauchten Kühlmitttel übertragen werden kann.

In einer bevorzugten Ausführungsform der vorliegenden Erfindung wird die aus dem Destillationsschritt (b) erhaltene, überwiegend Glykol enthaltende Fraktion (b2) anschließend mindestens einem der nachfolgend beschriebenen Schritte (c) bis (h) unterworfen.

Insbesondere beträgt in dem so erhaltenen Glykol der Gehalt an
- Ameisensäure bevorzugt nicht mehr als 250, besonders bevorzugt nicht mehr als 125, ganz besonders bevorzugt nicht mehr als 50, insbesondere nicht mehr als 25 und speziell nicht mehr als 10 Gew.ppm,
- Essigsäure bevorzugt nicht mehr als 500, besonders bevorzugt nicht mehr als 300, ganz besonders bevorzugt nicht mehr als 200, insbesondere nicht mehr als 100 und speziell nicht mehr als 50 Gew.ppm,
- Glykolsäure bevorzugt nicht mehr als 500, besonders bevorzugt nicht mehr als 300, ganz besonders bevorzugt nicht mehr als 200, insbesondere nicht mehr als 100 und speziell nicht mehr als 50 Gew.ppm,
- Dioxan bevorzugt nicht mehr als 500, besonders bevorzugt nicht mehr als 300, ganz besonders bevorzugt nicht mehr als 200, insbesondere nicht mehr als 100, speziell nicht mehr als 50 und sogar nicht mehr als 25 Gew.ppm,
- Formaldehyd bevorzugt nicht mehr als 250, besonders bevorzugt nicht mehr als 125, ganz besonders bevorzugt nicht mehr als 50, insbesondere nicht mehr als 25 und speziell nicht mehr als 10 Gew.ppm, und/oder
- Acetaldehyd bevorzugt nicht mehr als 250, besonders bevorzugt nicht mehr als 125, ganz besonders bevorzugt nicht mehr als 50, insbesondere nicht mehr als 25 und speziell nicht mehr als 10 Gew.ppm.

### (c) Strippen eines glykolhaltigen Gemischs mit einem Gas

Schritt (c) ist dann besonders bevorzugt, wenn das Glykol trotz der vorhergehenden Destillation einen Anteil an Leichtsiedern enthält, besonders aldehydische oder saure Verbindungen, beispielsweise Formaldehyd und/oder Acetaldehyd und deren höheren Oligomere, Glyoxal, Ameisensäure, Essigsäure, Oxalsäure, Glykolsäure und/oder Glyoxylsäure.

Dazu wird ein inertes Gas durch die glykolhaltige Zusammensetzung geleitet, mit dem leichtsiedende Inhaltsstoffe durch Strippen entfernt oder zumindest abgereichert werden.

Die kann beispielsweise in einem Rührkessel oder einer Blasensäule erfolgen, im Falle einer Blasensäule in Gleich- oder bevorzugt im Gegenstrom. Das inerte Gas wird durch eine Tauchung, beispielsweise eine Ringleitung oder Fritte durch die flüssige Zusammensetzung geleitet. Am Ausgang des Reaktors wird bevorzugt ein Schaumbrecher installiert, besonders bei hohen Volumenströmen des inerten Gases.

Erfolgt die Strippung in einer Kolonne, so kann diese mit Füllkörpern oder Packungen bestückt sein.

Zur Verringerung der Viskosität der glykolhaltigen Zusammensetzung kann die Flüssigkeit auf bis zu 100 °C erwärmt werden, bevorzugt bis zu 80, besonders bevorzugt bis zu 60 °C.

Bei dem inerten Gas handelt es sich bevorzugt um Argon oder ein stickstoffhaltiges Gas, besonders bevorzugt Argon, Stickstoff oder ein Gemisch aus Luft und Stickstoff (Magerluft), ganz besonders bevorzugt Stickstoff. Eingeleitet werden beispielsweise 0,1 - 10, bevorzugt 0,2 - 8 und besonders bevorzugt 0,5 - 5 m³/m³h, bezogen auf das Volumen des flüssigen Gemisches.

In einer bevorzugten Ausführungsform der vorliegenden Erfindung wird als Strippstrom zumindest dann Wasserdampf eingesetzt, wenn nach der Strippung noch eine Wasserabtrennung erfolgt. Somit ist Wasserdampf als Strippstrom in den Schritten (a) und/oder (b) bevorzugt und in Schritt (c) dann, wenn dieser vor Durchlaufen der Schritte (a) und (b) durchgeführt wird.

### (d) Behandlung eines glykolhaltigen Gemischs mit einem Feststoff

Schritt (d) ist dann besonders bevorzugt, wenn das Glykol trotz der vorhergehenden Destillation einen Anteil an Leichtsiedern enthält, besonders saure Verbindungen, beispielsweise Ameisensäure, Essigsäure, Oxalsäure, Glykolsäure und/oder Glyoxylsäure, besonders aber höhere organische Säuren.

Schritt (d) ist ebenfalls dann bevorzugt, wenn das Glykol anorganische Bestandteile enthält, besonders Silikate, Borate, Molybdate und/oder Phosphate.

Bei dem Feststoff kann es sich um eine sauren oder basischen, bevorzugt basischen Feststoff handeln.

Beispiele für derartige saure Feststoffe sind
- Natürliche Tonminerale: Kaolinit, Bentonit, Attapulgit, Montmorillonit, Clarit, Fuller Erde, Zeolithe (X, Y, A, H-ZSM usw.), kationenausgetauschte Zeolithe, Tone, Kieselerde, Quartzsand, Aluminiumoxide, Diatomeenerde.
- lonentauscherharze
- Metalloxide und -sulfide: ZnO, CdO, Al₂O₃, CeO₂, ThO₂, TiO₂, ZrO₂, SnO₂, PbO, As₂O₅, Bi₂O₃, Sb₂O₅, V₂O₅, Cr₂O₃, MoO₃, WO₃, CdS, ZnS
- Metallsalze: MgSO₄ , CaSO₄, SrSO₄ , BaSO₄, CuSO₄, ZnSO₄, CdSO₄, Al₂(SO₄)₃, FeSO₄, Fe₂(SO₄)₃, CoSO₄, NiSO₄, Cr₂(SO₄)₃, KHSO₄, K₂SO₄, (NH₄)₂SO₄, Zn(NO₃)₂, Ca(NO₃)₂, Bi(NO₃)₃, Fe(NO₃)₃, CaCO₃, BPO₄, AlPO₄, CrPO₄, FePO₄, Cu₃(PO₄)₂, Zn₃(PO₄)₂, Mg₃(PO₄)₂, Ti₃(PO₄)₄, Zr₃(PO₄)₄, Ni₃(PO₄)₂, AgCl, CuCI, CaCl₂, AlCl₃, TiCl₄, SnCl₄, CaF₂, BaF₂, AgClO₄, Mg(ClO₄)₂,
- Gemischte Oxide: SiO₂-Al₂O₃, SiO₂-TiO₂, SiO₂-SnO₂, SiO₂-ZrO₂, SiO₂-BeO, SiO₂-MgO, SiO₂-CaO, SiO₂-SrO, SiO₂-ZnO, SiO₂-Ga₂O₃, SiO₂-Y₂O₃, SiO₂-La₂O₃, SiO₂-MoO₃, SiO₂-WO₃, SiO₂-V₂O₅, SiO₂-ThO₂, Al₂O,-MgO, Al₂O₃-ZnO, Al₂O₃-CdO, Al₂O₃ -B₂O₃, Al₂O₃-ThO₂, Al₂O₃-TiO₂, Al₂O₃-ZrO₂, Al₂O₃-V₂O₅, Al₂O₃-MoO₃, Al₂O₃-WO₃, Al₂O₃-Cr₂O₃, Al₂O₃-Mn₂O₃, Al₂O₃-Fe₂O₃, Al₂O₃-Co₃O₄, Al₂O₃-NiO,TiO₂-CuO, TiO₂-MgO, TiO₂-ZnO, TiO₂-CdO, TiO₂-ZrO₂, TiO₂-SnO₂, TiO₂-Bi₂O₃, TiO₂-Sb₂O₅, TiO₂-V₂O₅, TiO₂-Cr₂O₃, TiO₂-MoO₃, TiO₂-WO₃, TiO₂-Mn₂O₃, TiO₂-Fe₂O₃, TiO₂-Co₃O₄, TiO₂-NiO, ZrO₂-CdO, ZnO-MgO, ZnO-Fe₂O₃,MoO₃-CoO-Al₂O₃, MoO₃-NiO- Al₂O₃, TiO₂-SiO₂-MgO, MoO₃-Al₂O₃-MgO, Heteropolysäuren.

Noch bevorzugter ist der saure Feststoffkatalysator ausgewählt aus der Gruppe bestehend aus SiO₂, Al₂O₃, TiO₂, ZrO₂, B₂O₃, ZnO₂, Nb₂O₅ oder Mischungen davon.

Besonders bevorzugt wird der saure Feststoffkatalysator aus der Gruppe bestehend aus Silikaten, Aluminiumoxid, Silico-Aluminaten und Zeolithen ausgewählt.

Insbesondere ist der saure Feststoffkatalysator ein Molekularsieb.

Der durchschnittliche Porendurchmesser solcher Molekularsiebe liegt bei 0,1 bis 1 nm (1 bis 10 Ä), vorzugsweise bei 0,1 bis 0,6, noch bevorzugter bei 0,2 bis 0,5 nm.

Solche Molekularsiebe sind Aluminosilikate mit einem Siliciumdioxid-Aluminiumoxid-Verhältnis (SiO₂/Al₂O₃) von 1 : 0,1 bis 1 : 5, vorzugsweise von 1 : 0,2 bis 1 : 3 und noch bevorzugter von 1 : 0,2 bis 1 : 1, insbesondere 1 : 0,5.

Die ungefähre chemische Zusammensetzung solcher Aluminosilikate ist

[(K₂O)ₓ (Na₂O)_{y}] • Al₂O₃• 2 SiO₂ • 9/2 H₂O

mit
x von 0 bis 1, bevorzugt von 0 bis 0,7, besonders bevorzugt von 0 bis 0,5, ganz besonders bevorzugt 0,
y von 0 bis 1, bevorzugt von 0,3 bis 1, besonders bevorzugt von 0,5 bis 1, ganz besonders bevorzugt 1,
worin x + y = 1.

Beispiele für basische Feststoffe sind neben basischem Aluminiumoxid auch (Erd)alkalimetall carbonate und -hydrogencarbonate, wie Kalziumcarbonat, Magnesiumcarbonat, Lithiumcarbonat, Natriumcarbonat, Kaliumcarbonat, Kalziumhydrogencarbonat, Magnesiumhydrogencarbonat, Lithiumhydrogencarbonat, Natriumhydrogencarbonat und Kaliumhydrogencarbonat.

Unter diesen sind die Carbonate bevorzugt, besonders bevorzugt die Alkalimetallcarbonate, besonders bevorzugt Natriumcarbonat, Natriumhydrogencarbonat und Kaliumcarbonat, ganz besonders bevorzugt sind Natriumcarbonat und Natriumhydrogencarbonat.

Die Abtrennung von Säuren erfolgt weiterhin bevorzugt über Durchleiten des glykolhaltigen Gemisches über basische lonentauscher, die Abtrennung von Basen über Durchleiten des glykolhaltigen Gemisches über saure lonentauscher.

Saure lonentauscher sind zumeist solche, die Carboxyl- oder Sulfonsäuregruppen, bevorzugt Carbonsäuregruppen in einer Polymermatrix, häufig eine Polymermatrix auf Basis Polystyrol, Poly(meth)acrylaten oder Poly(meth)acrylsäure tragen. Unter den sauren Ionentauschern sind die schwach sauren lonentauscher bevorzugt, insbesondere solche, deren Acidität durch Carboxylgruppen bestimmt wird. Ganz besonders solche auf Basis Poly(meth)acrylsäure.

Bei basischen Ionentauschern ist eine quartäre Ammoniumgruppe oder eine Aminogruppe an eine Polymermatrix gebunden. Quartäre Ammoniumgruppen sind beispielsweise Trimethylammoniumgruppen (-N⁺(CH₃)₃) oder Hydroxyethyl dimethylammoniumgruppen (-N⁺(CH₃)₂(-CH₂-CH₂-OH)). Aminogruppen können primär, sekundär oder tertiär sein, bevorzugt primär oder tertiär, besonders bevorzugt tertiär.

Bevorzugt wird die glykolhaltige Zusammensetzung kontinuierlich über eine Festbettschüttung des jeweiligen Feststoffs in einem Rohrreaktor geleitet, bevorzugt sind zwei Rohrreaktoren parallel geschaltet, damit ein Reaktor genutzt werden kann während in dem anderen Reaktor der Feststoff regeneriert wird.

Denkbar, wenn auch weniger bevorzugt, ist die Zugabe eines Feststoffs zur glykolhaltigen Zusammensetzung in einem Rührreaktor, wobei der Feststoff anschließend per Filtration wieder aus der glykolhaltigen Zusammensetzung entfernt wird. Ebenfalls denkbar ist die Fixierung des Feststoffs in einem Korb oder Sieb, was einerseits eine Durchströmung des Feststoffs und andererseits dessen leichte Abtrennung gewährleistet.

Zur Verringerung der Viskosität der glykolhaltigen Zusammensetzung kann die Flüssigkeit auf bis zu 100 °C erwärmt werden, bevorzugt bis zu 80, besonders bevorzugt bis zu 60 °C.

### (e) Behandlung eines glykolhaltigen Gemischs mit Aktivkohle

Schritt (e) ist dann besonders bevorzugt, wenn das Glykol trotz der vorhergehenden Destillation einen Anteil an Leichtsiedern enthält, besonders aldehydische Verbindungen, beispielsweise Formaldehyd und/oder Acetaldehyd und deren höheren Oligomere, Glyoxal und/oder Glyoxylsäure. Diese können dann in Schritt (e) an Aktivkohle adsorbiert werden.

Die Adsorption wird im bevorzugt kontinuierlich und an Aktivkohle in einer Festbettschüttung durchgeführt.

Unter Festbett wird eine Schüttschicht von Aktivkohle verstanden, die im wesentlichen in Ruhe bleibt, während die glykolhaltige Zusammensetzung durch das Festbett fließt.

Bevorzugt ist ein Verfahren, bei dem die kontinuierliche Adsorption in einer oder mehreren Säulen, insbesondere in einer oder zwei Säulen, durchgeführt wird, die mit Aktivkohle gefüllt sind.

Die Aktivkohle kann pflanzlichen oder tierischen Ursprungs sein und beliebige geometrische Form aufweisen, beispielsweise Splitt, Granulat, Kugeln, Tabletten oder Stränge, bevorzugt Splitt, Granulat oder Stränge und besonders bevorzugt Splitt oder Granulat.

Denkbar ist auch der Einsatz von Aktivkohle in Form von Pulver und dessen nachträgliche Absiebung oder Filtration, beispielsweise über ein feinporiges Filter oder ein Filtrierhilfsmittel.

Bevorzugt liegt beim erfindungsgemäßen Verfahren die Aktivkohle in einer Teilchengrößenverteilung vor, bei der mittlere Teilchendurchmesser größer 300 µm, bevorzugt größer 400 µm, insbesondere größer 500 µm vorliegt. Diese Teilchengrößen eignen sich für ein kontinuierliches Verfahren besonders, da die Aktivkohle leicht von der glykolhaltigen Zusammensetzung getrennt gehalten werden kann. Die Aktivkohle liegt typischerweise in granulierter Form vor. Besonders bevorzugt weisen 80 Gew.%, besonders bevorzugt 90 Gew.%, insbesondere 95 Gew% der Aktivkohle eine Teilchengrößen zwischen 350 µm und 1800 µm, insbesondere zwischen 420 µm und 1700 µm, auf.

Bevorzugt wird Aktivkohle verwendet, die eine hohe spezifische Oberfläche aufweist (> 600 m²/g, bevorzugt > 800 m²/g, insbesondere wird Aktivkohle mit einer spezifischen Oberfläche (nach DIN 66131) von 500 bis 2000 m²/g, bevorzugt 500 bis 1800 m²/g, besonders bevorzugt 900 bis 1100 m²/g eingesetzt.

Das Porenvolumen (nach DIN 66134) beträgt bevorzugt von 0,05 bis 1,0 cm³/g, bevorzugt 0,10 bis 0,95 cm³/g.

Die Dichte beträgt wird im Allgemeinen von 400 g/l bis 500 g/l.

Zur Verringerung der Viskosität der glykolhaltigen Zusammensetzung kann die Flüssigkeit auf bis zu 100 °C erwärmt werden, bevorzugt bis zu 80, besonders bevorzugt bis zu 60 °C.

Es kann sinnvoll sein, den Austrag aus der Stufe (e) einer Filtration zu unterwerfen, falls mit dem Austrag Aktivkohle aus dem Festbett mitgerissen wird.

### (f) Membranfiltration eines glykolhaltigen Gemischs

Schritt (f) wird besonders dann durchgeführt, um einen Restgehalt von Wasser aus dem Glykol zu entfernen oder um Glykol von oligomeren Glykolen, Polymeren und/oder höheren organischen Säuren abzutrennen.

Zur Wasserentfernung kann beispielsweise das wasserhaltige Glykol mit einem Druck von 3 bis 80 bar, bevorzugt 4 bis 60 bar über eine osmotische Membran mit Porenöffnungen bis zu 12 Ä, bevorzugt bis zu 10 Å aufgetrennt werden.

Das dabei erhaltene Solvent ist wasserangereichert, das Solut dementsprechend wasserabgereichert. Auf diesem Wege der Membranfiltration, besonders der Umkehrosmose, kann ein Wassergehalt bis zu 5 Gew% im Glykol, bevorzugt bis zu 3 Gew% nochmals um mindestens 50, bevorzugt mindestens 70 und besonders bevorzugt mindestens 80 % verringert werden.

Zur Abtrennung von Glykol von oligomeren Glykolen und/oder höheren organischen Säuren wird die Zusammensetzung mit einem Druck von 3 bis 100 bar, bevorzugt 4 bis 80 bar, besonders bevorzugt 5 bis 60 bar über eine osmotische Membran mit Porenöffnungen bis zu 30 Ä, bevorzugt bis zu 25 Ä aufgetrennt. Das dabei erhaltene Solvent ist glykolangereichert, im Solut sind die oligomeren Glykole bzw. höheren organischen Säuren angereichert.

Auf diesem Wege kann im Solvent ein Gehalt an oligomeren Glykolen bis zu 2 Gew% und/oder ein Gehalt an höheren organischen Säuren bis zu 1 Gew% um mindestens 50, bevorzugt mindestens 70 und besonders bevorzugt mindestens 80 % verringert werden.

Als Membranen eignen sich besonders polymere oder keramische Membranen, besonders bevorzugt Polymermembranen.

Die Filtration kann bevorzugt über Mikro- oder Ultrafiltration, besonders bevorzugt über Ultrafiltration erfolgen. Zur Abgrenzung wird eine Ausschlussgrenze von 100 nm angesehen: Bei einer Ausschlussgrenze von 100 nm oder mehr handeltes sich um eine Mikrofiltration, darunter (Ausschussgrenze 2 bis 100 nm) um Ultrafiltration.

Eine bevorzugte Ausführungsform der vorliegenden Erfindung stellt ein Verfahren zur Reinigung gebrauchter, wäßriger glykolhaltiger Kühlmittel dar, umfassend mindestens die Schritte
(a) destillative Abtrennung von leichter als Glykol siedenden Inhaltsstoffen,
(b) Destillation von Glykol,
   sowie darüberhinaus noch mindestens einen der Schritte
(d) Behandlung eines glykolhaltigen Gemischs mit einem sauren oder basischen, bevorzugt basischen Feststoff und/oder
(e) Behandlung eines glykolhaltigen Gemischs mit Aktivkohle.

Eine besonders bevorzugte Ausführungsform der vorliegenden Erfindung stellt ein Verfahren zur Reinigung gebrauchter, wäßriger glykolhaltiger Kühlmittel dar, umfassend mindestens die Schritte
(a) destillative Abtrennung von leichter als Glykol siedenden Inhaltsstoffen,
(b) Destillation von Glykol und
(d) Behandlung eines glykolhaltigen Gemischs mit einem sauren oder basischen, bevorzugt basischen Feststoff.

Eine weitere besonders bevorzugte Ausführungsform der vorliegenden Erfindung stellt ein Verfahren zur Reinigung gebrauchter, wäßriger glykolhaltiger Kühlmittel dar, umfassend mindestens die Schritte
(a) destillative Abtrennung von leichter als Glykol siedenden Inhaltsstoffen,
(b) Destillation von Glykol und
(e) Behandlung eines glykolhaltigen Gemischs mit Aktivkohle.

Bevorzugt werden die oben beschriebenen Reinigungsschritte in der Reihenfolge wie folgt durchgeführt:
- (a) - (b) - (d)
- (b) - (a) - (d)
- (a) - (b) - (e)
- (b) - (a) - (e)
- (a) - (b) - (d) - (e)
- (b) - (a) - (d) - (e)
- (a) - (b) - (e) - (d)
- (b) - (a) - (e) - (d)

Denkbar, wenn auch weniger bevorzugt sind die folgenden Reihenfolgen:
- (a) - (b) - (c)
- (b) - (a) - (c)
- (a) - (b) - (d) - (c)
- (b) - (a) - (d) - (c)
- (a) - (b) - (c) - (d)
- (b) - (a) - (c) - (d)

Es stellt einen Vorteil der vorliegenden Erfindung dar, dass bereits hergestellte Glykole in gebrauchten Kühlmitteln statt wie bisher entsorgt durch das beschriebene Verfahren bevorzugt als Kühlmittelkomponente wiederverwendet werden können.

Dadurch kann auf die neue Herstellung von Glykolen aus Ethylenoxid bzw. Propylenoxid zumindest teilweise verzichtet werden, wodurch weniger fossile Quellen verbraucht werden.

Dies stellt somit eine Verringerung der mit der Herstellung von Glykolen aus fossilen Quellen verbundenen Emissionen, beispielsweise Stickoxid- und Schwefeloxid-Emissionen und insbesondere der Kohlenstoffdioxid-Emissionen, bevorzugt bestimmt als Carbon-Footprint oder als Ökobilanz, besonders bevorzugt gemäß DIN EN ISO 14021, DIN EN ISO 14067, hier besonders die Ausgabe 2019-02, DIN EN ISO 14044, hier besonders die Ausgabe 2006 + A1:2018 und/oder DIN EN ISO 14040, hier besonders die Ausgabe 2009-11, indem man in Kühlmitteln das eingesetzte Glykol zumindest teilweise durch solches Glykol ersetzt, das aus dem oben genannten Aufarbeitungsverfahren erhalten worden ist.

Zur Bestimmung der Ökobilanz werden dabei bevorzugt die DIN EN ISO 14040, hier besonders die Ausgabe 2009-11 und/oder DIN EN ISO 14044, hier besonders die Ausgabe 2006 + A1:2018 zugrundegelegt.

Zur Bestimmung des Carbon-Footprint wird dabei bevorzugt DIN EN ISO 14067, hier besonders die Ausgabe 2019-02 zugrundegelegt.

Ein weiterer Gegenstand der vorliegenden Erfindung ist ein Verfahren zur Verringerung der Emissionen, insbesondere der Kohlenstoffdioxid-Emissionen, bevorzugt bestimmt als Carbon-Footprint oder Ökobilanz, besonders bevorzugt gemäß DIN EN ISO 14021, DIN EN ISO 14067, hier besonders die Ausgabe 2019-02, DIN EN ISO 14044, hier besonders die Ausgabe 2006 + A1:2018 und/oder DIN EN ISO 14040, hier besonders die Ausgabe 2009-11, in dem man gebrauchte, wäßrige glykolhaltige Kühlmittel einem Reinigungsverfahren unterwirft, umfassend mindestens die Schritte
(a) destillative Abtrennung von leichter als Glykol siedenden Inhaltsstoffen,
(b) Destillation von Glykol,
   sowie darüberhinaus noch mindestens einen der Schritte
(c) Strippen eines glykolhaltigen Gemischs mit einem Gas, bevorzugt einem Inertgas,
(d) Behandlung eines glykolhaltigen Gemischs mit einem sauren oder basischen, bevorzugt basischen Feststoff,
(e) Behandlung eines glykolhaltigen Gemischs mit Aktivkohle, und/oder
(f) Membranfiltration eines glykolhaltigen Gemischs,
und man das aus diesem Verfahren erhaltene Glykol zur Herstellung neuer Kühlmittel verwendet.

Ein weiterer Gegenstand der vorliegenden Erfindung ist ein Verfahren zur Verringerung der Emissionen, insbesondere der Kohlenstoffdioxid-Emissionen, bevorzugt bestimmt als Carbon-Footprint oder Ökobilanz, besonders bevorzugt gemäß DIN EN ISO 14021, DIN EN ISO 14067, hier besonders die Ausgabe 2019-02, DIN EN ISO 14044, hier besonders die Ausgabe 2006 + A1:2018 und/oder DIN EN ISO 14040, hier besonders die Ausgabe 2009-11, in dem man
- zunächst gebrauchte wässrige Kühlmittel, bevorzugt aus Kraftfahrzeugen und/oder ortsfesten Motoren oder Generatoren, dezentral sammelt, bevorzugt von Werkstätten und/oder Wartungsfirmen,
- die so gesammelten gebrauchten wässrigen Kühlmittel sammelt, vereinigt und zu einem zentralen Ort transportiert und dort diese gebrauchten, wäßrigen glykolhaltigen Kühlmittel einem Reinigungsverfahren unterwirft, umfassend mindestens die Schritte
   (a) destillative Abtrennung von leichter als Glykol siedenden Inhaltsstoffen,
   (b) Destillation von Glykol,
      sowie darüberhinaus noch mindestens einen der Schritte
   (c) Strippen eines glykolhaltigen Gemischs mit einem Gas, bevorzugt einem Inertgas,
   (d) Behandlung eines glykolhaltigen Gemischs mit einem sauren oder basischen, bevorzugt basischen Feststoff,
   (e) Behandlung eines glykolhaltigen Gemischs mit Aktivkohle, und/oder
   (f) Membranfiltration eines glykolhaltigen Gemischs,
   und man das aus diesem Verfahren erhaltene Glykol zur Herstellung neuer Kühlmittel verwendet.

Durch den geringeren Einsatz von Ausgangsstoffen aus fossilen Quellen erreicht man dabei eine Verringerung der Emissionen, besonders der CO₂-Emissionen, beispielsweise bestimmt durch den Carbon Footprint, Ökobilanzierung oder gemäß DIN EN ISO 14021, DIN EN ISO 14067, hier besonders die Ausgabe 2019-02, DIN EN ISO 14044, hier besonders die Ausgabe 2006 + A1:2018 und/oder DIN EN ISO 14040, hier besonders die Ausgabe 2009-11.

Ein weiterer Gegenstand der vorliegenden Erfindung ist die Verwendung von Glykolen, erhalten aus einem der oben beschriebenen Verfahren, als gefrierpunktserniedrigende Komponente in Kühlmitteln, besonders für ortsfesten Anlagen, beispielsweise Brennstoffzellen, Aggregaten, Generatoren oder Windkraftanlagen sowie in Kraftfahrzeugen.

Da beim Sammeln und der Aufarbeitung der gebrauchten Kühlmittel in der Praxis keine vollständige Rückgewinnung des eingesetzten Glykols erreicht werden kann, wird eine vollständige Kreislaufwirtschaft hinsichtlich des Glykols praktisch nicht erreicht. Daher wird es in aller Regel erforderlich sein, das aus einem der oben beschriebenen Verfahren erhaltene Glykol mit einem frisch hergestellten Glykol zu vermischen.

Dieses frisch hergestellte Glykol kann dabei konventionell aus fossilen Quellen hergestellt werden, wie oben dargestellt, kann aber auch stattdessen oder zusätzlich aus nachwachsenden Quellen hergestellt werden.

Demzufolge ist ein weiterer Gegenstand der vorliegenden Erfindung ein Kühlmittel, enthaltend
- mindestens 40 Gew% Wasser (A)
- mindestens 30 Gew% Glykol (B),
   als Inhibitoren (C)
- (C1) optional mindestens eine anorganische Verbindung ausgewählt aus der Gruppe bestehend aus Silikaten, Boraten, Nitraten, Molybdaten und Phosphaten
- (C2a) optional Benzoesäure als aromatische Monocarbonsäure
- (C2b) optional mindestens eine aliphatischen Monocarbonsäure,
- (C3) optional mindestens eine organische Dicarbonsäuren, die 4 bis 20 Kohlenstoffatome aufweist
- (C4) mindestens eine Azolverbindung, bevorzugt mindestens eine Triazolverbindung
- (D) optional mindestens eine anorganische Base
- (E) mindestens einen sonstigen Bestandteil, ausgewählt aus der Gruppe bestehend aus Hartwasserstabilisatoren, Entschäumer, Farbstoffe und Bitterstoffe
wobei
die Komponente (B) zumindest teilweise Monoethylenglykol und/oder Monopropylenglykol enthält, das aus einem der oben beschriebenen Verfahren erhalten worden ist.

In einer bevorzugten Ausführungsform enthält die Komponente (B) in diesem erfindungsgemäßen Kühlmittel
- mindestens 25 Gew%, bevorzugt mindestens 30, besonders bevorzugt mindestens 40, ganz besonders bevorzugt mindestens 50, insbesondere mindestens 60 Gew%, speziell 100 Gew% Monoethylenglykol und/oder Monopropylenglykol (B1), das aus einem der oben beschriebenen Verfahren erhalten worden ist und
- nicht mehr als 75 Gew%, bevorzugt nicht mehr als 70 Gew%, besonders bevorzugt nicht mehr als 60 Gew%, ganz besonders bevorzugt nicht mehr als 50, insbesondere nicht mehr als 40 Gew% und speziell 0 Gew% Monoethylenglykol und/oder Monopropylenglykol (B2), das direkt aus einem Herstellungsprozess der Umsetzung von Ethylenoxid bzw. Propylenoxid mit Wasser erhalten worden ist, wobei das Ethylenoxid bzw. Propylenoxid aus fossilen Quellen stammt, sowie optional
- Monoethylenglykol und/oder Monopropylenglykol (B3), das direkt aus einem Herstellungsprozess aus nachwachsenden Rohstoffen erhalten worden ist.

In einer bevorzugten Ausführungsform der vorliegenden Erfindung besteht die Komponente (B) vollständig aus Monoethylenglykol und/oder Monopropylenglykol (B1) ohne weitere Beimischung von Produkt aus fossilen Quellen oder nachwachsenden Rohstoffen.

In einer weiteren bevorzugten Ausführungsform der vorliegenden Erfindung besteht die Komponente (B) vollständig aus Monoethylenglykol und/oder Monopropylenglykol (B1) und Monoethylenglykol und/oder Monopropylenglykol (B3) aus nachwachsenden Rohstoffen ohne weitere Beimischung von Produkt (B2) aus fossilen Quellen.

In einer weiteren bevorzugten Ausführungsform der vorliegenden Erfindung besteht die Komponente (B) vollständig aus Monoethylenglykol und/oder Monopropylenglykol (B1) und Monoethylenglykol und/oder Monopropylenglykol (B2) aus fossilen Quellen ohne weitere Beimischung von Produkt (B3) aus nachwachsenden Rohstoffen.

Mit dem Begriff "direkt" ist dabei gemeint, dass das jeweilige Glykol bei Einsatz in dem erfindungsgemäßen Kühlmittel zuvor nicht bereits in einem Kühlmittel verwendet worden ist, sondern als unmittelbares Verfahrensprodukt aus der industriellen Produktion des Glykols stammt.

Die typischen Kühlmittelbestandteile (C) bis (E) seien im Folgenden beschrieben:

### Inhibitoren (C)

Die Inhibitoren (C) wirken als Korrosionsinhibitoren gegen Metallkorrosion, beispielsweise von Eisenwerkstoffen, Aluminium, Buntmetallen oder Lot.

Die erfindungsgemäßen Zusammensetzungen enthalten
- (C1) optional mindestens eine anorganische Verbindung ausgewählt aus der Gruppe bestehend aus Silikaten, Boraten, Nitraten, Molybdaten und Phosphaten und organischen Kieselsäureestern
- (C2a) optional Benzoesäure als aromatische Monocarbonsäure
- (C2b) optional mindestens eine aliphatischen Monocarbonsäure
- (C3) optional mindestens eine organische Dicarbonsäure, die 4 bis 20 Kohlenstoffatome aufweist
- (C4) mindestens eine Azol, bevorzugt mindestens eine Triazolverbindung.

### Anorganische Inhibitoren (C1)

Bei den anorganischen Inhibitoren (C1) handelt es sich um Silikate, Borate, Nitrate, Molybdate oder Phosphate, oder deren Mischungen in Form ihrer freien Säuren oder ihrer Salze, besonders ihrer Alkalimetallsalze, besonders bevorzugt ihrer Natrium- oder Kaliumsalze. In welcher Form (protoniert oder als Salz) sie in den Zusammensetzungen, Superkonzentraten, Konzentraten oder Kühlmitteln vorliegen hängt von dem jeweiligen pKₛ-Wert der Verbindung und der Zusammensetzung sowie dem pH des jeweiligen Milieus ab, der sich durch die Menge der Base (D) einstellt.

Die anorganischen Silikate wirken überwiegend als Inhibitor der Korrosion von Aluminium und werden meist als Alkalimetallsalze oder seltener als Magnesium-, Calcium- oder Aluminiumsalze eingesetzt, bevorzugt als Natrium- oder Kaliumsalze.

Die Silikate sind bevorzugt ausgewählt aus der Gruppe bestehend aus Orthosilikaten (SiO₄⁴⁻), Metasilikaten (SiO₃²⁻), und Pyrosilikaten (Si₂O₇⁶⁻), besonders bevorzugt handelt es sich um Metasilikate (SiO₃²⁻), ganz besonders bevorzugt um Natriummetasilikat (Na₂SiO₃) oder Kaliummetasilikat (K₂SiO₃), insbesondere um Natriummetasilikat (Na₂SiO₃).

Wenn die erfindungsgemäße feste Zusammensetzung mindestens ein anorganisches Silikat oder einen organischen Kieselsäureester enthält, so wird in einer bevorzugten Ausführungsform zusätzlich zu dem Silikat mindestens ein Silicophosphonat zudosiert, wie es beschrieben ist in EP 4015596 bzw. in der WO 2022/043303 für Kieselsäureester.

Bevorzugt handelt es sich bei dem Silicophosphonat um eine Verbindung der allgemeinen Formel 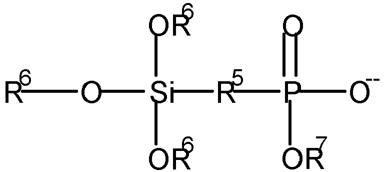 worin
R⁵ ein zweibindiger organischer Rest ist, bevorzugt eine 1,ω-Alkylen Gruppe mit 1 bis 6, bevorzugt 1 bis 4 Kohlenstoffatomen, besonders bevorzugt Methylen, 1,2-Ethylen, 1,2-Propylen, 1,3-Propylen oder 1,4-Butylen, ganz besonders bevorzugt 1,2-Ethylen oder 1,3-Propylen und insbesondere 1,2-Ethylen,
R⁶ unabhängig voneinander Wassertoff, C₁- bis C₄-Alkyl oder Hydroxy-C₂- bis C₄-Alkyl, bevorzugt Wasserstoff, Methyl, Ethyl, n-Propyl, iso-Propyl, n-Butyl, iso-Butyl, sec-Butyl oder tert-Butyl, 2-Hydroxyethyl oder 2-Hydroxypropyl, besonders bevorzugt Wasserstoff, Methyl, Ethyl oder Propyl,
und R⁷ ist C₁- bis C₄-Alkyl, bevorzugt Methyl, Ethyl, n-Propyl oder n-Butyl, besonders bevorzugt Methyl, Ethyl oder n-Butyl, ganz besonders bevorzugt Methyl oder Ethyl und insbesondere Methyl.

Die Silicophosphonate können als freie Säure oder als Alkalimetallsalz eingesetzt werden, bevorzugt als Natrium- oder Kaliumsalz und besonders bevorzugt als Natriumsalz.

Die Borate werden bevorzugt als Natriumtetraborat (Borax) oder als Kaliumtetraborat eingesetzt, besonders bevorzugt als Natriumtetraborat.

Die Nitrate werden als Alkali- oder Erdalkalimetallnitrate eingesetzt, bevorzugt als Natriumnitrat, Kaliumnitrat oder Magnesiumnitrat, bevorzugt als Natriumnitrat oder Kaliumnitrat, besonders bevorzugt als Natriumnitrat.

Die Phosphate werden als freie Säure (H₃PO₄), als Hydrogenphosphat, Dihydrogenphosphat oder Phosphat eingesetzt, bevorzugt als Natrium oder Kaliumsalz.

Denkbar ist auch der Einsatz der entsprechenden Diphosphate, Triphosphate oder Oligophosphate, bevorzugt werden sie jedoch als monomere Phosphate eingesetzt.

Bevorzugt ist der Einsatz als freie Säure (H₃PO₄), Dinatriumhydrogenphosphat oder Trinatriumphosphat.

Ester der Orthokieselsäure sind Verbindungen der Formel

Si(OR¹)₄

worin
R¹ ist ein organischer Substituent mit 1 bis 6 Kohlenstoffatomen, beispielsweise ein linearer oder verzweigter, vorzugsweise ein linearer Alkylsubstituent mit 1 bis 6 Kohlenstoffatomen oder ein aromatischer Substituent mit 6 Kohlenstoffatomen, besonders bevorzugt ein Alkylsubstituent mit 1 bis 4 Kohlenstoffatomen und ganz besonders bevorzugt ein Alkylsubstituent mit 1 oder 2 Kohlenstoffatomen.

Alkoxyalkylsilane sind weniger bevorzugt und sowohl der Alkoxysubstituent als auch die Alkylgruppe umfassen einen linearen oder verzweigten, vorzugsweise einen linearen Alkylsubstituenten mit 1 bis 6 Kohlenstoffatomen, besonders bevorzugt einen Alkylsubstituenten mit 1 bis 4 Kohlenstoffatomen und ganz besonders bevorzugt einen Alkylsubstituenten mit 1 oder 2 Kohlenstoffatomen.

Typische Beispiele für Verbindungen (D) sind Tetraalkoxysilane, vorzugsweise Tetramethoxysilan und Tetraethoxysilan, und Alkoxyalkylsilane, vorzugsweise Triethoxymethylsilan, Dithoxydim-Thylsilan, Ethoxytrimethylsilan, Trimethoxymethylsilan, Dimethoxydimethylsilan und Methoxy-Trimethylsilan. Bevorzugt werden Tetraalkoxysilane, besonders bevorzugt Tetramethoxysilan und Tetraethoxysilan, wobei Tetraethoxysilan ganz besonders bevorzugt wird.

Bevorzugt handelt es sich bei den Komponenten (C1) um mindestens eine Verbindung ausgewählt aus der Gruppe bestehend aus Silikaten, Boraten, Nitraten oder Phosphaten, besonders bevorzugt um mindestens eine Verbindung ausgewählt aus der Gruppe bestehend aus Silikaten, Nitraten oder Phosphaten.

### (C2a) Aromatische Monocarbonsäure

Bei der optionalen aromatischen Monocarbonsäure handelt es sich bevorzugt um Benzoesäure, die als freie Säure oder besonders bevorzugt in Form ihres Alkalimetallsalzes eingesetzt werden kann, ganz besonders bevorzugt bevorzugt als Natrium benzoat.

In einer bevorzugten Ausführungsform der vorliegenden Erfindung ist keine aromatische Monocarbonsäure anwesend.

### (C2b) Aliphatische Monocarbonsäuren

Bei den aliphatischen Monocarbonsäuren handelt es sich um organische aliphatische Alkan- oder Alkencarbonsäuren. Diese werden, ausreichende Wasserlöslichkeit vorausgesetzt, in Kühlmitteln häufig als Korrosionsinhibitoren gegen die Korrosion von Eisenwerkstoffen eingesetzt. Bevorzugt weisen diese aliphatischen Monocarbonsäuren 5 bis 12 Kohlenstoffatome auf, besonders bevorzugt 6 bis 10 und ganz besonders bevorzugt 8, 9 oder 10.

Typische derartige Monocarbonsäuren sind Pentansäure, 2,2-Dimethylpropansäure, Hexansäure, 2,2-Dimethylbutansäure, Octansäure, 2-Ethylhexansäure, n-Nonansäure, Isononansäure, Decansäure, Undecansäure und Dodecansäure, sowie deren Isomerengemische, insbesondere 2-Ethylhexansäure, n-Nonansäure und Isononansäure-Isomerengemische.

Es stellt jedoch eine mögliche Ausführungsform dar, die aliphatischen Monocarbonsäuren in Form ihrer Alkalimetallsalze, bevorzugt in Form ihrer Lithium-, Natrium- oder Kaliumsalze, besonders bevorzugt in Form ihrer Natrium- oder Kaliumsalze anstelle der freien Säure einzusetzen.

### (C3) 4 bis 20 Kohlenstoffatome aufweisende organische Dicarbonsäure

Bei den 4 bis 20 Kohlenstoffatome aufweisenden organischen Dicarbonsäuren handelt es sich um lineare oder verzweigte Alkandicarbonsäuren, bevorzugt lineare Alkan- oder Alkendicarbonsäuren, besonders bevorzugt lineare Alkandicarbonsäuren, besonders bevorzugt mit 5 bis 14 und ganz besonders bevorzugt mit 6 bis 12 Kohlenstoffatomen.

Bevorzugt sind die Dicarbonsäuren (C3) ausgewählt aus der Gruppe bestehend aus Bernsteinsäure, Maleinsäure, Fumarsäure, Glutarsäure, Adipinsäure, Pimelinsäure (Heptandisäure), Azelainsäure (Nonandisäure), Sebacinsäure (Dekandisäure), Undekandisäure, Dodekandisäure, sowie Alkyl- und Alkenylbernsteinsäuren und -glutarsäuren wie 2-Methylbutandisäure, 2-ethyl-3-methylbutandisäure, 2-Ethylpentandisäure, 2-Dodecylbutandisäure, 2-Dodecenylbutandisäure, 2-Phenylbutandisäure, 2-(p-Methyl phenyl) butandisäure, 2,2-Dimethylbutandisäure, 2,3,4-Trimethylpentandisäure, 2,2,3-Trimethylpentandisäure, Glutaconsäure (Pent-2-endisäure), Itaconsäure, Hex-2-endisäure, Hex-3-endisäure, 5-Methyl-hex-2-endisäure und 2,3-Diemethyl-pent-2-endisäure.

Unter diesen sind die Dicarbonsäuren bevorzugt, die 6 bis 12 Kohlenstoffatome aufweisen, besonders bevorzugt unter diesen die Alkandicarbonsäuren, die 6 bis 12 Kohlenstoffatome aufweisen, ganz besonders bevorzugt die linearen Alkandicarbonsäuren, die 6 bis 12 Kohlenstoffatome aufweisen.

Insbesondere bevorzugt sind als Dicarbonsäuren (D3) Adipinsäure, Sebacinsäure, Azelainsäure und Dodecandicarbonsäure.

### (C4) Azolverbindung

Unter Azolderivaten (C4) werden im Rahmen dieser Schrift fünfgliedrige heterocyclische Verbindungen mit 2 oder 3 Heteroatomen aus der Gruppe Stickstoff und Schwefel, welche kein oder maximal ein Schwefelatom in den Ring eingebaut enthalten und welche optional einen aromatischen oder gesättigten sechsgliedrigen Anellanten tragen können.

Diese fünfgliedrigen heterocyclischen Verbindungen (Azolderivate) enthalten als Heteroatome üblicherweise zwei N-Atome und kein S-Atom, 3 N-Atome und kein S-Atom oder ein N-Atom und ein S-Atom.

Bevorzugte Gruppen der genannten Azolderivate sind anellierte Imidazole und anellierte 1,2,3-Triazole der allgemeinen Formel 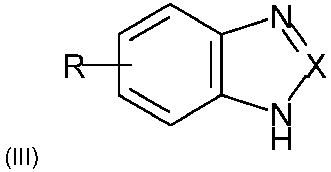 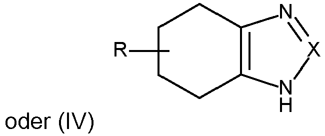 in denen die Variable
R Wasserstoff oder einen C₁- bis C₁₀-Alkylrest, insbesondere Methyl oder Ethyl, bedeutet und die Variable X ein Stickstoffatom oder die Gruppierung C-H bezeichnet.

Typische und bevorzugte Beispiele für Azolderivate der allgemeinen Formel (III) sind Benzimidazol (X = C-H, R = H), Benzotriazol (X = N, R = H) und Tolutriazol (Tolyltriazol) (X = N, R = CH₃). Ein typisches Beispiel für ein Azolderivat der allgemeinen Formel (IV) ist hydriertes 1,2,3-Tolutriazol (Tolyltriazol) (X = N, R = CH₃).

Eine weitere bevorzugte Gruppe der genannten Azolderivate sind Benzthiazole der allgemeinen 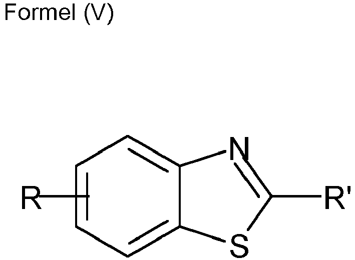 in der
die Variable R die oben genannte Bedeutung hat und
die Variable R' Wasserstoff, einen C₁- bis C₁₀-Alkylrest, insbesondere Methyl oder Ethyl, oder insbesondere eine Mercaptogruppe (-SH) bezeichnet. Denkbar, wenn auch weniger bevorzugt kann R' auch ein Carboxyalkylrest der Formel -(CₘH₂ₘ)-COOR" sein, wobei m für eine Zahl von 1 bis 4 steht und R" Wasserstoff oder C₁- bis C₁₀-Alkyl, insbesondere Methyl oder Ethyl, oder C₆- bis C₁₂-Aryl bedeutet. Beispiele dafür sind (2-Benzothiazylthio)-essigsäure, (2-Benzothiazylthio)-essigsäure ester, 3-(2-Benzothiazylthio)-propionsäure oder 3-(2-Benzothiazylthio)-propionsäure ester. Für den Fall, daß diese Verbindungen als Säure eingesetzt werden, so zählen sie nicht zu den erfindungsgemäß ausgeschlossenen Carbonsäuren. Ein typisches Beispiel für ein Azolderivat der allgemeinen Formel (V) ist 2-Mercaptobenzthiazol.

Weiterhin werden nicht-anellierte Azolderivate der allgemeinen Formel (VI) 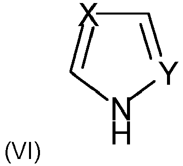 in der die Variablen
X und Y zusammen zwei Stickstoffatome oder
ein Stickstoffatom und eine Gruppierung C-H bezeichnen,
beispielsweise 1H-1,2,4-Triazol (X = Y = N) oder bevorzugt Imidazol (X = N, Y = C-H).

Ganz besonders bevorzugt werden für die vorliegende Erfindung als Azolderivate Benzimidazol, Benzotriazol, Tolutriazol, hydriertes Tolutriazol oder Mischungen hieraus, insbesondere Benzotriazol oder Tolutriazol, speziell Tolutriazol.

Die genannten Azolderivate sind kommerziell verfügbar oder nach gängigen Methoden herstellbar. Hydrierte Benzotriazole wie hydriertes Tolutriazol sind ebenfalls gemäß DE-A 1 948 794 zugänglich und auch kommerziell verfügbar.

Bevorzugt sind die Azole ausgewählt aus der Gruppe bestehend aus Benzotriazol, Tolutriazol, (2-Benzothiazylthio)-essigsäure, 3-(2-Benzothiazylthio)-propionsäure und 2-Mercaptobenzthiazol.

### (D) Anorganische Base

Der pH-Wert der Gefrierschutzmittel beim Endanwender liegt üblicherweise im Bereich von 4 bis 11,5, vorzugsweise 5 bis 10, insbesondere 6 bis 9.

Um diesen pH-Wert einzustellen werden bei dem Herstellungsverfahren der Kühlmittel aus einer konzentrierten Vorstufe auf einer beliebigen Stufe mindestens eine anorganische Base (D) zugegeben. Dabei kann die mindestens eine anorganische Base in der erfindungsgemäßen Zusammensetzung, im Superkonzentrat oder im Konzentrat enthalten sein oder bei der Herstellung des Superkonzentrats aus der erfindungsgemäßen Zusammensetzung durch Vermischen mit der Komponente (A) und/oder (B), bei der Herstellung des Konzentrats aus dem Superkonzentrat durch Vermischen mit der Komponente (A) und/oder (B) oder bei der Herstellung des Kühlmittels aus dem Konzentrat durch Vermischen mit der Komponente (A) und/oder (B) zugegeben werden.

Daher enthalten die erfindungsgemäßen Zusammensetzungen optional eine Menge an anorganischer Base, die bei entsprechender Verdünnung im Kühlmittel diesen gewünschten pH-Wert einstellt. Dafür enthalten die erfindungsgemäßen Zusammensetzungen bevorzugt Alkalimetallhydroxid, besonders bevorzugt festes Lithium-, Natrium- oder Kaliumhydroxid, gegebenenfalls auch in Form von wässriger Lithium-, Natron- oder Kalilauge.

Weniger bevorzugt sind Carbonate oder Hydrogencarbonate des Lithium, Natrium oder Kalium.

Bevorzugte Alkalimetalle sind Natrium und Kalium.

In einer bevorzugten Ausführungsform ist zumindest ein Teil der anorganischen Base, bevorzugt die gesamte erforderliche anorganische Base bereits in der erfindungsgemäßen Zusammensetzung enthalten. Dies hat einerseits den Vorteil, daß die Base auf keiner späteren Herstellungsstufe mehr zugegeben werden muß und somit die Gefahr einer Fehldosierung entfällt, andererseits liegen die zugegebenen Säuren dadurch in Form ihrer zumeist leichter kristallisierenden Alkalimetallsalzform vor, was die Formulierung der festen erfindungsgemäßen Zusammensetzung als Feststoff erleichtert.

### (E) Sonstige Bestandteile, ausgewählt aus der Gruppe bestehend aus Hartwasserstabilisatoren, Entschäumer, Farbstoffe und Bitterstoffe

Als weitere übliche Hilfsmittel können die erfindungsgemäßen Zusammensetzung in üblichen geringen Mengen noch Entschäumer (in der Regel in Mengen von 0,003 bis 0,008 Gew.-% in dem fertig verdünnten Kühlmittel) sowie aus Gründen der Hygiene und der Sicherheit im Falle eines Verschluckens Bitterstoffe (z. B. vom Typ Denatoniumbenzoat) und Farbstoffe enthalten.

Ferner können die Zusammensetzung einen oder mehrere Hartwasserstabilisatoren auf Basis von Polyacrylsäure, Polymaleinsäure, Acrylsäure-Maleinsäure-Copolymeren, Polyvinylpyrrolidon, Polyvinylimidazol, Vinylpyrrolidon-Vinylimidazol-Copolymeren und/oder Copolymeren aus ungesättigten Carbonsäuren und Olefinen enthalten. Der Anteil in der Zusammensetzung werden so gewählt, daß nach entsprechender Verdünnung die Menge in dem fertig verdünnten Kühlmittel bis zu 1 Gew.-% beträgt.

### Konzentrate, Superkonzentrate

Um die zu transportierenden Volumina zu verringern, werden zumeist nicht die stark wasserhaltigen Kühlmittel, sondern stattdessen Konzentrate vertrieben, in denen der Wasseranteil weggelassen oder stark reduziert wird. Die Kühlmittel werden vom Endanwender aus den Konzentraten durch Wasserzugabe hergestellt.

Zur weiteren Verringerung der zu transportierenden Volumina werden oftmals sogenannte Superkonzentrate zentral hergestellt, in denen nicht nur der Wasser-, sondern zusätzlich auch der Glykolanteil weggelassen oder stark reduziert ist. Aus diesen Superkonzentraten werden dann erst regional von Formulierern durch Abmischung mit Glykolen die Konzentrate hergestellt.

Erfindungsgemäß wesentlich ist, dass diese zur Abmischung eingesetzten Glykole die Forderung erfüllen, zumindest teilweise aus der Aufreinigung gebrauchter Kühlmittelzusammensetzungen erhalten worden zu sein.

Ein weiterer Gegenstand der vorliegenden Erfindung sind Kühlmittelkonzentrate, enthaltend
- nicht mehr als 15, bevorzugt nicht mehr als 10 und besonders bevorzugt nicht mehr als 5 Gew% Wasser (A)
- mindestens ein Alkylenglykol, Alkylenglykolmonoalkylether oder Glycerin (B) als Inhibitoren (C)
- (C1) optional mindestens eine anorganische Verbindung ausgewählt aus der Gruppe bestehend aus Silikaten, Boraten, Nitraten, Molybdaten und Phosphaten
- (C2a) optional Benzoesäure als aromatische Monocarbonsäure
- (C2b) optional mindestens eine aliphatischen Monocarbonsäure,
- (C3) optional mindestens eine organische Dicarbonsäuren, die 4 bis 20 Kohlenstoffatome aufweist
- (C4) mindestens eine Azol-, bevorzugt mindestens eine Triazolverbindung
- (D) optional mindestens eine anorganische Base
- (E) mindestens einen sonstigen Bestandteil, ausgewählt aus der Gruppe bestehend aus Hartwasserstabilisatoren, Entschäumer, Farbstoffe und Bitterstoffe
in dem
die Komponente (B) zumindest teilweise Monoethylenglykol und/oder Monopropylenglykol enthält, bevorzugt Monoethylenglykol, das zumindest teilweise aus der Aufreinigung gebrauchter Kühlmittelzusammensetzungen erhalten worden ist.

Ein weiterer Gegenstand der vorliegenden Erfindung sind Kühlmittelsuperkonzentrate, enthaltend
- nicht mehr als 15, bevorzugt nicht mehr als 10 und besonders bevorzugt nicht mehr als 5 Gew% Wasser (A)
- mindestens ein Alkylenglykol, Alkylenglykolmonoalkylether oder Glycerin (B) als Inhibitoren (C)
- (C1) optional mindestens eine anorganische Verbindung ausgewählt aus der Gruppe bestehend aus Silikaten, Boraten, Nitraten, Molybdaten und Phosphaten
- (C2a) optional Benzoesäure als aromatische Monocarbonsäure
- (C2b) optional mindestens eine aliphatischen Monocarbonsäure,
- (C3) optional mindestens eine organische Dicarbonsäuren, die 4 bis 20 Kohlenstoffatome aufweist
- (C4) mindestens eine Azol, bevorzugt mindestens eine Triazolverbindung
- (D) optional mindestens eine anorganische Base
- (E) mindestens einen sonstigen Bestandteil, ausgewählt aus der Gruppe bestehend aus Hartwasserstabilisatoren, Entschäumer, Farbstoffe und Bitterstoffe
in dem
die Komponente (B) zumindest teilweise Monoethylenglykol und/oder Monopropylenglykol enthält, bevorzugt Monoethylenglykol, das zumindest teilweise aus der Aufreinigung gebrauchter Kühlmittelzusammensetzungen erhalten worden ist.

Ein weiterer Gegenstand der vorliegenden Erfindung ist ein Verfahren zur Herstellung der oben genannten Kühlmittelkonzentrate aus den oben genannten Kühlmittelsuperkonzentraten, in dem man ein Kühlmittelsuperkonzentrat mit der entsprechenden Menge mindestens eines Alkylenglykols, Alkylenglykolmonoalkylethers oder Glycerin (B) versetzt, mit der Maßgabe dass man als Komponente (B) zumindest teilweise Monoethylenglykol und/oder Monopropylenglykol einsetzt, bevorzugt Monoethylenglykol, das zumindest teilweise aus der Aufreinigung gebrauchter Kühlmittelzusammensetzungen erhalten worden ist.

Es stellt eine bevorzugte Ausführungsform der vorliegenden Erfindung dar, dieses erfindungsgemäße Kühlmittel zum teilweisen oder vollständigen Ersatz gebrauchter Kühlmittel in ortsfesten Anlagen, beispielsweise Brennstoffzellen, Aggregaten, Generatoren oder Windkraftanlagen sowie in Kraftfahrzeugen, bevorzugt beim Endanwender (sogenannte aftermarket Verwendung), zu verwenden. Dabei setzt der Endanwender das erfindungsgemäße Kühlmittel ein, um im Kraftfahrzeug verbrauchtes Kühlmittel zu ergänzen oder zumindest teilweise zu ersetzen um so die Gebrauchseigenschaften des Kühlmittels zu verbessern.

### Beispiele

### Beispiel: Destillation eines gebrauchten Kühlmittels

Das gebrauchte Kühlmittel wurde diskontinuierlich aus einem Doppelmantelgefäß (Volumen 1,6 Liter) als Vorlage mit aufgesetzter Destillationskolonne (Länge 1m, Durchmesser 30 mm, Sulzer DX-Packungen, entsprechend ca. 20 theoretischen Böden) mit Kondensator (0,15 m²) und Rücklaufteiler destilliert. Die Destillatabnahme erfolgte aus dem Rücklaufteiler am Kopf unterhalb des Kondensators.

Die Sumpftemperatur wurde im Verlauf der Destillation von 55 °C auf bis zu 216 °C gesteigert, der Kopfdruck der Destillationskolonne wurde auf 100 mbar (absolut) eingestellt.

Während der ersten ca. 3,5 Std wurde Wasser bei einem Rücklaufverhältnis von 1 : 2 (Sekunden Rücklauf: Sekunden Abnahme) bei einer Kopftemperatur von ca. 48 °C abdestilliert, nach Anstieg der Kopftemperatur auf ca. 129 °C wurde das Rücklaufverhältnis auf 2 : 2 geändert, für weitere ca. 50 Minuten die Zwischenfraktion und anschließend für etwa 2 Stunden die Hauptfraktion gesammelt (Kopftemperatur 129 - 132 °C).

In der Destillationsvorlage verblieb nach einer Gesamtdauer von ca. 6 Stunden ein Hochsiederrückstand von ca. 3,3 Gew%.

Die Ausbeute an Monoethylenglykol (bezogen auf den gaschromatographisch ermittelten Gehalt im Ausgangsmaterial) betrug 49 % in der Hauptfraktion bzw. 91 % bezogen auf die Summe aus Übergangs- und Hauptfraktion.

### Analysedaten

### Analyse per Gaschromatographie in Gew%, wenn nicht anders angegeben

### Wassergehalt per Karl Fischer-Titration

| | Ausgangsmaterial | Übergangsfraktion | Hauptfraktion |
|---|---|---|---|
| Wasser | 50,27 | 0,60 | 0,12 |
| Monoethylenglykol | 46,4 | 99,8 (*) | 99,9 (*) |
| Diethylenglykol | 0,2 | < 0,01 (*) | < 0,01 (*) |
| Polyethylenglykol | < 0,1 | 0,08 (*) | 0,05 (*) |
| Formaldehyd | | 12 ppm | 5 ppm |
| Acetaldehyd | | 1 ppm | 1 ppm |
| Propionaldehyd | | < 1 ppm | < 1 ppm |
| Andere Aldehyde (überwiegend Glyoxal) | | ca. 10 ppm | ca. 10 ppm |
| pH | 7,4 | 5,3 | |
| Aussehen | klar, rötlich | klar, farblos | klar, farblos |

| | | | |
|---|---|---|---|
| (*): Flächen% im Gaschromatogramm | | | |

Der Gehalt an Ameisensäure, Glycolsäure und Essigsäure in den Fraktionen betrug jeweils
< 10 ppm
APHA-Farbzahl: < 5

Man sieht, dass mit der beschriebenen Destillation aus gebrauchten Kühlmitteln Wasser und oligomere Ethylenglykole, sowie Aldehyde und Säuren gut abgetrennt werden können.

Die Hauptfraktion war als solche ohne weiteres zur Formulierung von Kühlmittel einsetzbar, die Übergangsfraktion sollte zur Abtrennung von Formaldehyd noch mindestens einem der Schritte (c) bis (f) unterzogen werden.

### Beispiel: Berechnung des Product Carbon Footprint (PCF) gemäß ISO 14067:2018

Zum Vergleich werden zwei Modelle der Entsorgung bzw. Verwertung von gebrauchten Kühlmitteln am Ende ihrer Nutzungsdauer (sogenannte "cradle-to-grave" Betrachtung) verglichen und die unten angegeben Nachhaltigkeitskennzahlen per Simulation bestimmt. Den Modellen liegt jeweils zugrunde, dass das gebrauchte Kühlmittel z.B. in Werkstätten gesammelt wird, beispielsweise im Rahmen einer regulären Inspektion eines Kfz, und anschließend entsorgt (Modell 1) bzw. wiedergewonnen (Modell 2) wird.

### Alternative 1 (Lineares Modell, Vergleich)

Es wurde angenommen, das wässrige Kühlmittel werde am Ende seiner Nutzungsdauer in einer üblichen deutschen Kläranlage entsorgt und das enthaltene Ethylenglykol sei 100 % biologisch abbaubar, d.h. der enthaltene organische Kohlenstoff (TOC, total organic carbon) wird vollständig in CO₂ überführt.

Der enthaltene organische Kohlenstoff TOC im wässrigen Kühlmittel ist in die Rechnung mit 0,2 kg C/kg Lösung eingegangen. Aufgrund interner Quellen wurde weiterhin angenommen, dass bei der Abwasserbehandlung 2 kWh pro kg TOC aufgewendet wurden. Dieser Energiebedarf sowie die CO₂-Emissionen bei der Herstellung des Kühlmittels (gemäß Deutschem Strommix) wurden in die Berechnung einbezogen. Die CO₂-Äquivalente pro kg Kühlmittel werden aus Gründen der Vergleichbarkeit bezogen auf ein Kühlmittelkonzentrat mit hohem Anteil an Ethylenglykol.

Nicht in der Simulation berücksichtigt wurde der Kohlenstofffußabdruck PCF für den Transport des zugrundeliegenden Kühlmittels von der Produktion über die Abfüllung zum Verbraucher (Werkstätten).

### Ergebnis:

| | |
|---|---|
| CO₂-Äquivalente | 2,4 kg CO₂ |

### Alternative 2 (zirkuläres Modell)

Es wurde angenommen, das wässrige Kühlmittel falle am Ende seiner Nutzungsdauer als 30%ige wässrige Lösung (abzüglich 10% handhabungsbedingter Verluste, die wie in Modell 1 einer Kläranlage zugeführt werden) in der Werkstatt an, werde zur Aufarbeitung transportiert und dort einer destillativen Aufarbeitung unterworfen.

In der Destillation fallen drei Fraktionen sowie ein Rückstand an:
Fraktion 1: Rein wässrige Fraktion, die ohne CO₂-Freisetzung in der Kläranlage behandelt wird
Fraktion 2: Mischfraktion Wasser/Ethylenglykol (zur Verbrennung)
Fraktion 3: redestilliertes Ethylenglykol
Rückstand: Ethylenglykol, anorganische Bestandteile, organische Hochsieder (zur Verbrennung) und Verluste

Die Destillationsausbeute betrug dabei 0,23 kg Ethylenglykol/kg Kühlmittel, für die Destillation im kontinuierlichen Betrieb müssen 5,45 kg Dampf/kg redestilliertes Ethylenglykol aufgewandt werden. Auch hier wurden die CO₂-Emissionen dieses Energieaufwandes sowie der Herstellung des Kühlmittels (gemäß Deutschem Strommix) in die Berechnung einbezogen.

Auch hier wurde der Kohlenstofffußabdruck PCF für den Transport des zugrundeliegenden Kühlmittels von der Produktion über die Abfüllung zum Verbraucher (Werkstätten) nicht in der Simulation berücksichtigt.

### Ergebnis:

| | |
|---|---|
| CO₂-Äquivalente | 1,7 kg CO₂ |

### Nachhaltigkeitskennzahlen

Ein exemplarisches Kühlmittelkonzentrat, das nach diesen beiden Modellen erhalten wird, weist in einer Simulation folgende Nachhaltigkeitskennzahlen auf:

| | Alternative 1 (Lineares Modell) | Alternative 2 (zirkuläres Modell) | Einheit |
|---|---|---|---|
| PCF (ISO 14067:2018) | 2,4 | 1,7 | kg CO₂ Äquivalente |
| Fossile Ressourcennutzung | 42,35 | 16,14 | MJ/kg |
| Landnutzung | 39,58 | 39,42 | Pt/kg |
| Acidifizierung (saurer Regen) | 5,06 × 10⁻³ | 4,31 × 10⁻³ | mol H+ eq./kg |
| Photochemische Ozonbildung | 2,51 × 10⁻³ | 1,96 × 10⁻³ | kg NMVOC eq./kg |

Die Kennzahlen wurden mittels einer LCA-Software (Life Cycle Assessment) GaBi dabei nach folgenden Methoden bestimmt:
- Product Carbon Footprint (PCF): gemäß ISO 14067:2018, basierend auf ISO 14040:2006 und 14044:2006 die Abschätzung des Lebenszyklus, abgeglichen mit dem GHG Protocol Product Standard (WRI & WBCSD, 2011).
- Fossile Ressourcennutzung (Energieträger): Gemäß CML 2002 Modell, Guinée, J.B. (Ed.), Gorrée, M., Heijungs, R., Huppes, G., Kleijn, R., de Koning, A., Van Oers, L., Wegener Sleeswijk, A., Suh, S.,. Udo de Haes, H.A, De Bruijn, J.A., Van Duin R., Huijbregts, M.A.J. (2002). Handbook on Life Cycle Assessment: Operational Guide to the ISO Standards. p. 63-95, Series: Eco-efficiency in industry and science. Kluwer Academic Publishers. Dordrecht. Oers, L.F.C.M., van & Koning, A., de & Guinée, J.B. & Huppes, G. (2002): Abiotic resource depletion in LCA: improving characterisation factors for abiotic depletion as recommended in the new Dutch LCA Handbook. Delft: Ministry of Transport, Public Works and Water Management.
- Landnutzung: Bodenqualitätsindex basierend auf dem LANCA-Modell, Beck, T., Bos, U., Wittstock, B., Baitz, M., Fischer, M., Sedlbauer, K. (2010). 'LANCA Land Use Indicator Value Calculation in Life Cycle Assessment - Method Report', Fraunhofer Institute for Building Physics.
- Acidifizierung (saurer Regen): J. Seppälä, M. Posch, M. Johansson and J.-P. Hettelingh (2006): Country-Dependent Characterization Factors for Acidification and Terrestrial Eutrophication Based on Accumulated Exceedance as an Impact Category Indicator. Int J LCA 11 (6): 403 - 416. M. Posch, J. Seppälä, J,-P.-Hettelingh, M. Johansson, M. Margni; O. Jolliet (2008): The role of atmospheric dispersion models and ecosystem sensitivity in the determination of characterization factors for acidifying and eutrophying emissions in LCIA. Int J Life Cycle Assess (2008) 13: 477-486.
- Photochemische Ozonbildung (Sommersmog): LOTOS-EUROS Modell, R. van Zelm, M.A.J. Huijbregts, H.A. den Hollander, H.A. van Jaarsveld, F.J. Sauter, J. Struijs, H.J. van Wijnen, D. van de Meent (2008): European characterization factors for human health damage of PM10 and ozone in life cycle impact assessment. Atmospheric Environment 42: 441-453.

## Patentansprüche

1. Verfahren zur Reinigung gebrauchter, wäßriger glykolhaltiger Kühlmittel, in dem man
- zunächst in einem Schritt (a) von dem gebrauchten, wäßrigen glykolhaltigen Kühlmittel destillativ leichter als Glykol siedende Inhaltsstoffe bei einem Druck von 50 mbar bis Normaldruck und einer Temperatur von 50 bis 140 °C abtrennt und
- anschließend in einem Schritt (b) aus dem Destillationsrückstand des Schrittes (a) das Glykol bei einem Druck von 50 mbar bis Normaldruck und einer Temperatur von 50 bis 140 °C abdestilliert.

2. Verfahren gemäß Anspruch 1, **dadurch gekennzeichnet, daß** Schritt (b) zwei Rektifikationskolonnen umfasst, in deren erster die überwiegend Wasser und Glykol enthaltende Fraktion (b1) über Kopf abgetrennt wird, der Destillationsrückstand aus Glykol und Hochsieder dann in die zweite Rektifikationskolonne geführt wird, in der die Glykolfraktion (b2) über Kopf abgetrennt wird und der Destillationssumpf die Hochsiederfraktion (b3) bildet,
wobei die Rektifikationskolonnen jeweils von 10 bis 40 theoretische Trennböden aufweisen.

3. Verfahren gemäß Anspruch 1, **dadurch gekennzeichnet, daß** Schritt (b) in einer einzelnen Rektifikationskolonne ausgeführt wird, in der Wasser und Glykol als Kopffraktion (b1), die Glykolfraktion (b2) im Seitenabzug und die Hochsieder im Sumpf (b3) abgenommen wird,
wobei die Rektifikationskolonne von 10 bis 60 theoretische Trennböden aufweist.

4. Verfahren zur Reinigung gebrauchter, wäßriger glykolhaltiger Kühlmittel, umfassend mindestens die Schritte
(a) destillative Abtrennung von leichter als Glykol siedenden Inhaltsstoffen,
(b) Destillation von Glykol,sowie darüberhinaus noch mindestens einen der Schritte
(c) Strippen eines glykolhaltigen Gemischs mit einem Gas, bevorzugt einem Inertgas,
(d) Behandlung eines glykolhaltigen Gemischs mit einem sauren oder basischen, bevorzugt basischen Feststoff,
(e) Behandlung eines glykolhaltigen Gemischs mit Aktivkohle, und/oder
(f) Membranfiltration eines glykolhaltigen Gemischs.

5. Verfahren gemäß Anspruch 4, **dadurch gekennzeichnet, daß** das Gas in Schritt (c) ausgewählt ist aus der Gruppe bestehend aus Luft, Magerluft, Wasserdampf, Stickstoff und Argon.

6. Verfahren gemäß Anspruch 5, **dadurch gekennzeichnet, daß** der Schritt (c) bei einer erhöhten Temperatur bis 100 °C durchgeführt wird.

7. Verfahren gemäß Anspruch 5 oder 6, **dadurch gekennzeichnet, daß** in Schritt (c) mindestens ein Aldehyd aus dem glykolhaltigen Gemisch abgetrennt wird.

8. Verfahren gemäß Anspruch 4, **dadurch gekennzeichnet, daß** der saure oder basische Feststoff in Schritt (d) ausgewählt ist aus der Gruppe bestehend aus
- Natürliche Tonminerale: Kaolinit, Bentonit, Attapulgit, Montmorillonit, Clarit, Fuller Erde, Zeolithe (X, Y, A, H-ZSM usw.), kationenausgetauschte Zeolithe, Tone, Kieselerde, Quartzsand, Aluminiumoxide, Diatomeenerde
- lonentauscherharze
- Metalloxide und -sulfide: ZnO, CdO, Al₂O₃, CeO₂, ThO₂, TiO₂, ZrO₂, SnO₂, PbO, As₂O₅, Bi₂O₃, Sb₂O₅, V₂O₅, Cr₂O₃, MoO₃, WO₃, CdS, ZnS
- Metallsalze: MgSO₄ , CaSO₄, SrSO₄ , BaSO₄, CuSO₄, ZnSO₄, CdSO₄, Al₂(SO₄)₃, FeSO₄, Fe₂(SO₄)₃, CoSO₄, NiSO₄, Cr₂(SO₄)₃, KHSO₄, K₂SO₄, (NH₄)₂SO₄, Zn(NO₃)₂, Ca(NO₃)₂, Bi(NO₃)₃, Fe(NO₃)₃, CaCO₃, BPO₄, AlPO₄, CrPO₄, FePO₄, Cu₃(PO₄)₂, Zn₃(PO₄)₂, Mg₃(PO₄)₂, Ti₃(PO₄)₄, Zr₃(PO₄)₄, Ni₃(PO₄)₂, AgCl, CuCI, CaCl₂, AlCl₃, TiCl₄, SnCl₄, CaF₂, BaF₂, AgClO₄, Mg(ClO₄)₂,
- Gemischte Oxide: SiO₂-Al₂O₃, SiO₂-TiO₂, SiO₂-SnO₂, SiO₂-ZrO₂, SiO₂-BeO, SiO₂-MgO, SiO₂-CaO, SiO₂-SrO, SiO₂-ZnO, SiO₂-Ga₂O₃, SiO₂-Y₂O₃, SiO₂-La₂O₃, SiO₂-MoO₃, SiO₂-WO₃, SiO₂-V₂O₅, SiO₂-ThO₂, Al₂O,-MgO, Al₂O₃-ZnO, Al₂O₃-CdO, Al₂O₃ -B₂O₃, Al₂O₃-Th0₂, Al₂O₃-TiO₂, Al₂O₃-ZrO₂, Al₂O₃-V₂O₅, Al₂O₃-MoO₃, Al₂O₃-WO₃, Al₂O₃-Cr₂O₃, Al₂O₃-Mn₂O₃, Al₂O₃-Fe₂O₃, Al₂O₃-Co₃O₄, Al₂O₃-NiO,TiO₂-CuO, TiO₂-MgO, TiO₃-ZnO, TiO₂-CdO, TiO₂-ZrO₂, TiO₂-SnO₂, TiO₂-Bi₂O₃, TiO₂-Sb₂O₅, TiO₂-V₂O₅, TiO₂-Cr₂O₃, TiO₂-MoO₃, TiO₂-WO₃, TiO₂-Mn₂O₃, TiO₂-Fe₂O₃, TiO₂-Co₃O₄, TiO₃-NiO, ZrO₂-CdO, ZnO-MgO, ZnO-Fe₂O₃,MoO₃-CoO-Al₂O₃, MoO₃-NiO- Al₂O₃, TiO₂-SiO₂-MgO, MoO₃-Al₂O₃-MgO, Heteropolysäuren
- basischem Aluminiumoxid
- (Erd)alkalimetall carbonate und -hydrogencarbonate und
- lonentauschern.

9. Verfahren gemäß Anspruch 8, **dadurch gekennzeichnet, daß** in Schritt (d) mindestens eine organische Carbonsäure aus dem glykolhaltigen Gemisch abgetrennt wird.

10. Verfahren gemäß Anspruch 8, **dadurch gekennzeichnet, daß** in Schritt (d) mindestens ein anorganischer Bestandteil aus dem glykolhaltigen Gemisch abgetrennt wird.

11. Verfahren gemäß Anspruch 4, **dadurch gekennzeichnet, daß** in Schritt (e) mindestens ein Aldehyd aus dem glykolhaltigen Gemisch abgetrennt wird.

12. Verfahren gemäß mindestens einem der vorstehenden Ansprüche, **dadurch gekennzeichnet, daß** das gebrauchte Kühlmittel wie folgt zusammengesetzt ist:
- Wasser: 50 bis 75 Gew%
- Glykol: 25 bis 45 Gew%
- höhere Glykololigomere: 0,1 bis 3 Gew%
- C₁-Abbauprodukte: 0,1 bis 2 Gew%
- C₂-Abbauprodukte: 0,1 bis 2 Gew%
- höhere organische Säuren und deren Abbauprodukte: 0,1 bis 5 Gew%
- anorganische Bestandteile: 0,1 bis 3 Gew%
- andere Bestandteile: bis zu 5 Gew%
mit der Maßgabe, dass die Summe immer 100 Gew% beträgt.

13. Verfahren zur Verringerung der Emissionen, insbesondere der Kohlenstoffdioxid-Emissionen, bevorzugt bestimmt als Carbon-Footprint oder Ökobilanz, besonders bevorzugt gemäß DIN EN ISO 14021, DIN EN ISO 14067, hier besonders die Ausgabe 2019-02, DIN EN ISO 14044, hier besonders die Ausgabe 2006 + A1:2018 und/oder DIN EN ISO 14040, hier besonders die Ausgabe 2009-11, in dem man gebrauchte, wäßrige glykolhaltige Kühlmittel einem Reinigungsverfahren unterwirft, umfassend mindestens die Schritte
(a) destillative Abtrennung von leichter als Glykol siedenden Inhaltsstoffen,
(b) Destillation von Glykol,
sowie darüberhinaus optional noch mindestens einen der Schritte
(c) Strippen eines glykolhaltigen Gemischs mit einem Gas, bevorzugt einem Inertgas,
(d) Behandlung eines glykolhaltigen Gemischs mit einem sauren oder basischen, bevorzugt basischen Feststoff,
(e) Behandlung eines glykolhaltigen Gemischs mit Aktivkohle, und/oder
(f) Membranfiltration eines glykolhaltigen Gemischs,
und man das aus diesem Verfahren erhaltene Glykol zur Herstellung neuer Kühlmittel verwendet.

14. Verfahren gemäß Anspruch 13, **dadurch gekennzeichnet, daß** man
- zunächst gebrauchte wässrige Kühlmittel, bevorzugt aus Kraftfahrzeugen und/oder ortsfesten Motoren, dezentral sammelt, bevorzugt von Werkstätten und/oder Wartungsfirmen,
- die so gesammelten gebrauchten wässrigen Kühlmittel sammelt, vereinigt und zu einem zentralen Ort transportiert und dort diese gebrauchten, wäßrigen glykolhaltigen Kühlmittel in ein Reinigungsverfahren gemäß Anspruch 13 führt.

15. Verwendung von Glykolen, erhalten aus einem Verfahren gemäß einem der Ansprüche 1 bis 14, als gefrierpunktserniedrigende Komponente in Kühlmitteln, besonders für ortsfesten Anlagen, beispielsweise Brennstoffzellen, Aggregaten, Generatoren oder Windkraftanlagen sowie in Kraftfahrzeugen oder zum Wärmemanagement in von elektronischen Geräten.

16. Kühlmittel, enthaltend
- mindestens 40 Gew% Wasser (A)
- mindestens 30 Gew% Glykol (B),
als Inhibitoren (C)
- (C1) optional mindestens eine anorganische Verbindung ausgewählt aus der Gruppe bestehend aus Silikaten, Boraten, Nitraten, Molybdaten und Phosphaten
- (C2a) optional Benzoesäure als aromatische Monocarbonsäure
- (C2b) optional mindestens eine aliphatischen Monocarbonsäure,
- (C3) optional mindestens eine organische Dicarbonsäuren, die 4 bis 20 Kohlenstoffatome aufweist
- (C4) mindestens eine Azolverbindung, bevorzugt mindestens eine Triazolverbindung
- (D) optional mindestens eine anorganische Base
- (E) mindestens einen sonstigen Bestandteil, ausgewählt aus der Gruppe bestehend aus Hartwasserstabilisatoren, Entschäumer, Farbstoffe und Bitterstoffe
wobei
die Komponente (B) zumindest teilweise Monoethylenglykol und/oder Monopropylenglykol enthält, das aus einem Verfahren gemäß einem der Ansprüche 1 bis 14 erhalten worden ist.
